# EUROPEAN PATENT APPLICATION

(11) **EP 4 563 163 A1**
(43) Date of publication of application: **04.06.2025**
(21) Application number: 23845580.2
(22) Date of filing: 26.07.2023
(51) Int. Cl.: A61K 47/68, C07D 491/22, A61K 31/4745, C07K 16/28, A61K 39/00, A61P 35/00

(54) **ANTIBODY-DRUG CONJUGATE AND USE THEREOF**

(30) Priority: 27.07.2022 CN 202210889771
(71) Applicant: Minghui Pharmaceutical (Hangzhou) Limited, Hangzhou, Zhejiang 310018 (CN); Minghui Pharmaceutical (Shanghai) Limited, Shanghai 201203 (CN)
(72) Inventor: ZHU, Liyuan, Shanghai 201203 (CN); CAO, Guoqing, Shanghai 201203 (CN); LI, Ao, Shanghai 201203 (CN); CHEN, Yile, Hangzhou, Zhejiang 310018 (CN); SHI, Junwei, Shanghai 201203 (CN); LIU, Bo, Hangzhou, Zhejiang 310018 (CN)
(74) Representative: SONN Patentanwälte GmbH & Co KG
(86) International application number: PCT/CN2023/109251
(87) International publication number: WO 2024/022372

(57) **Abstract**

The present disclosure relates to an antibody-drug conjugate and the use thereof. Specifically, provided is an antibody-drug conjugate as represented by formula I, or a stereoisomer thereof, a prodrug thereof, a pharmaceutically acceptable salt thereof or a pharmaceutically acceptable solvate thereof, Ab-(L-D)ₐ formula I.

## Description

The present disclosure is based on and claims the benefit of priority from CN Application No. 202210889771.5, filed on July 27, 2022, the disclosures of which are incorporated herein the present disclosure in its entirety.

### Technical Field

The present disclosure relates to the field of pharmaceutical technology, specifically relates to an antibody-drug conjugate and use thereof.

### Background Art

Antibody-drug Conjugate (ADC) is a novel type of therapeutic macromolecule, which combines the powerful killing power of small molecule drugs and the highly specific targeting of antibodies. It can be used to precisely deliver the drug to the tumor site or/and tumor cells by using the specificity of antibodies, avoiding the killing of normal cells in the body, thus reducing adverse reactions during the course of treatment. ADC consists of three components, namely antibody, drug and linker. When the antibody of ADC specifically binds to the specific antigen on the surface of tumor cells, the cell membrane of the tumor cell will be concave inwards, forming an endosome to swallow the ADC, the process of which is also known as internalization, the antibody or linker of the ADC will be degraded by some enzymes in the cell, thus releasing the drug, and then the released drug begins to act to kill the tumor cells.

B7-H3 (B7 homolog 3 protein), also known as CD276 or B7RP-2, is a kind of immune checkpoint molecule and belongs to B7 family of immunomodulatory proteins. B7-H3 has a low expression in normal tissues but is highly expressed in a variety of solid tumors, such as lung cancer, head and neck cancer, oesophageal cancer, ovarian cancer, breast cancer, prostate cancer, melanoma, and the like, and is associated with poor prognosis in a wide range of tumors. The use of B7-H3 as a therapeutic target may be an effective strategy for the treatment of cancers with high B7-H3 expression. A variety of B7-H3-targeted therapies are currently under development, including monoclonal antibodies, bispecific antibodies, CAR-T, antibody-drug conjugate (ADC), and the like, but there are no approved drugs on market yet. Among them, antibody-drug conjugate targetedly brings small molecule drugs (payloads) into target highly expressed tumor cells through the specific antibody of B7-H3, selectively reduces the off-target toxicity of small molecule drugs while retaining the tumor killing properties of small molecule drugs and has a greater potential to improve the benefit-risk ratio of anti-tumor therapy, which is expected to bring greater benefits to patients.

### Contents of the Present Disclosure

The present disclosure provides an antibody-drug conjugate represented by formula I, a stereoisomer thereof, a prodrug thereof, a pharmaceutically acceptable salt thereof, or a pharmaceutically acceptable solvate thereof. The antibody-drug conjugate shows excellent inhibitory activities against multiple tumor cell lines with different target expression levels and strong *in vivo* proliferation-inhibition effects in subcutaneous xenograft tumor models of human-derived tumor cells in nude mice, which has broad prospect in application.

For this purpose, in a first aspect of the present disclosure, the present disclosure provides an antibody-drug conjugate represented by formula I, a stereoisomer thereof, a prodrug thereof, a pharmaceutically acceptable salt thereof, or a pharmaceutically acceptable solvate thereof,

**Ab-(L-D)ₐ** **I**

Wherein, Ab is an antibody, such as a monoclonal antibody or an antigen-binding fragment thereof;
L is L¹-L²-L³-L⁴;
L¹ is 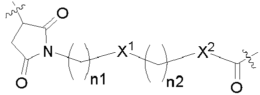 , and the carbonyl carbon end of L¹ is connected to L², and the alkyl carbon end of L¹ is connected to Ab;
   n1 is an integer selected from 1 to 5;
   n2 is an integer selected from 1 to 5;
   X¹ is a bond or O;
   X² is a bond or O;
L² is a bond or 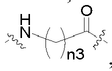 , and when L² is 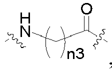 the amino end of L² is connected to L¹, and the carbonyl end of L² is connected to L³;
   n3 is an integer selected from 1 to 5;
L³ is an amino acid residue or a peptide residue consisting of 2 to 10 amino acid residues, and the amino end of L³ is connected to L² and the carbonyl end of L³ is connected to L⁴;
L⁴ is 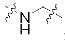 , and the amino end of L⁴ is connected to L³, and the carbon end of L⁴ is connected to D;
D is 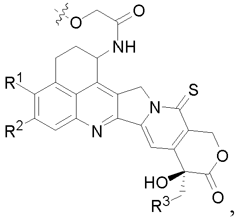 and the O end of D is connected to L⁴, wherein
R¹ is selected from the group consisting of hydrogen, deuterium, halogen, C1-C6 alkyl, deuterated C1-C6 alkyl, and halogenated C1-C6 alkyl;
R² is selected from the group consisting of hydrogen, deuterium, halogen, C1-C6 alkyl, deuterated C1-C6 alkyl, and halogenated C1-C6 alkyl;
R³ is selected from the group consisting of hydrogen, deuterium, halogen, C1-C6 alkyl, deuterated C1-C6 alkyl, and halogenated C1-C6 alkyl;
a is any number between 1 and 10.

In some embodiments, n1 is 1, 2 or 3. In some embodiments, n1 is 1. In some embodiments, n1 is 2. In some embodiments, n1 is 3. In some embodiments, n1 is 4. In some embodiments, n1 is 5.

In some embodiments, n2 is 1 or 2. In some embodiments, n2 is 1. In some embodiments, n2 is 2. In some embodiments, n2 is 3. In some embodiments, n2 is 4. In some embodiments, n2 is 5.

In some embodiments, n3 is 1, 2 or 3. In some embodiments, n3 is 2. In some embodiments, n3 is 1. In some embodiments, n3 is 3. In some embodiments, n3 is 4. In some embodiments, n3 is 5.

In some embodiments, L¹ is selected from the group consisting of 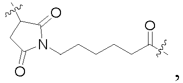 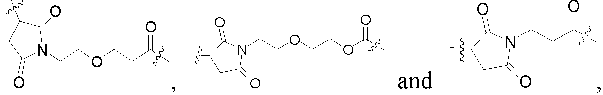 and the carbonyl carbon end of L¹ is connected to L², and the alkyl carbon end of L¹ is connected to Ab.

In some embodiments, L¹ is selected from the group consisting of 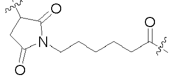 and 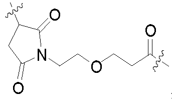 , and the carbonyl carbon end of L¹ is connected to L², and the alkyl carbon end of L¹ is connected to Ab.

In some embodiments, L¹ is 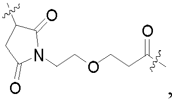 and the carbonyl carbon end of L¹ is connected to L², and the alkyl carbon end of L¹ is connected to Ab.

In some embodiments, L¹ is 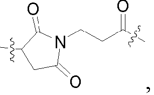 and the carbonyl carbon end of L¹ is connected to L², and the alkyl carbon end of L¹ is connected to Ab.

In some embodiments, L¹ is 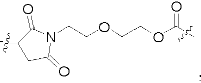 , and the carbonyl carbon end of L¹ is connected to L², and the alkyl carbon end of L¹ is connected to Ab.

**In** some embodiments, L³ is a peptide residue consisting of 2-4 (preferably 4) amino acid residues, with the amino end connected to L² and the carbonyl end connected to L⁴, preferably, the amino acid is selected from the group consisting of glycine, phenylalanine, valine, alanine, lysine, citrulline, serine, glutamic acid, and aspartic acid, and more preferably, the amino acid is selected from the group consisting of glycine and phenylalanine.

In some embodiments, L³ is glycine-glycine-phenylalanine-glycine (Gly-Gly-Phe-Gly), and the amino end is connected to L². and the carbonyl end is connected to L⁴.

In some embodiments, L³ is 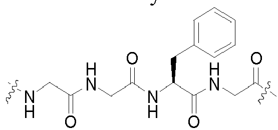 , and the amino end is connected to L², and the carbonyl end is connected to L⁴.

In some embodiments, R¹ is selected from the group consisting of hydrogen, halogen and C1-C6 alkyl.

In some embodiments, R¹ is selected from the group consisting of hydrogen, halogen and C1-C4 alkyl.

In some embodiments, R¹ is C1-C6 alkyl.

In some embodiments, R¹ is C1-C4 alkyl. In some embodiments, R¹ is selected from the group consisting of methyl, ethyl, n-propyl, isopropyl, n-butyl, isobutyl, sec-butyl, and tert-butyl. In some embodiments, R¹ is selected from the group consisting of methyl, ethyl, and n-propyl.

In some embodiments, R¹ is selected from the group consisting of fluorine, chlorine, bromine, and iodine.

In some embodiments, R¹ is selected from the group consisting of fluorine, chlorine, and bromine.

In some embodiments, R¹ is methyl.

In some embodiments, R² is selected from the group consisting of hydrogen, halogen and C1-C6 alkyl.

In some embodiments, R² is selected from the group consisting of hydrogen, halogen and C1-C4 alkyl.

In some embodiments, R² is C1-C6 alkyl. In some embodiments, R² is C1-C4 alkyl. In some embodiments, R² is selected from the group consisting of methyl, ethyl, n-propyl, isopropyl, n-butyl, isobutyl, sec-butyl, and tert-butyl. In some embodiments, R² is selected from the group consisting of methyl, ethyl, and n-propyl.

In some embodiments, R² is halogen. In some embodiments, R² is selected from the group consisting of fluorine, chlorine, bromine, and iodine. In some embodiments, R² is selected from the group consisting of fluorine, chlorine, and bromine.

In some embodiments, R² is fluorine. In some embodiments, R² is methyl.

In some embodiments, R³ is selected from the group consisting of hydrogen, halogen, and C1-C6 alkyl.

In some embodiments, R³ is selected from the group consisting of hydrogen, halogen, and C1-C4 alkyl.

In some embodiments, R³ is selected from the group consisting of fluorine, chlorine, bromine, and iodine. In some embodiments, R³ is selected from the group consisting of fluorine, chlorine, and bromine.

In some embodiments, R³ is C1-C6 alkyl.

In some embodiments, R³ is C1-C4 alkyl.

In some embodiments, R³ is selected from the group consisting of methyl, ethyl, n-propyl, isopropyl, n-butyl, isobutyl, sec-butyl, and tert-butyl. In some embodiments, R³ is selected from the group consisting of methyl, ethyl, and n-propyl.

In some embodiments, R³ is methyl.

In some embodiments, L is selected from the group consisting of: 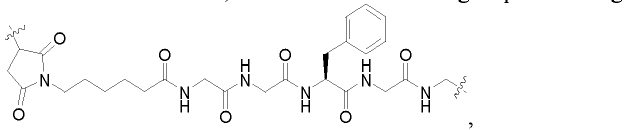 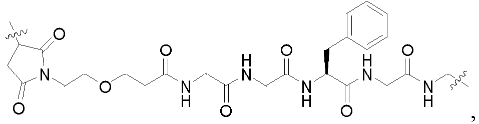 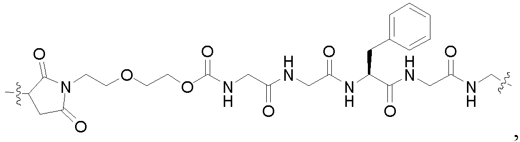 and 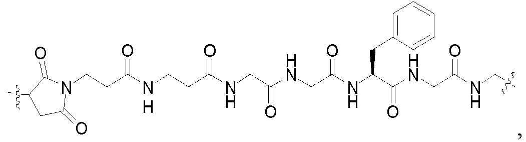 and the left carbon end of L is connected to Ab, and the right carbon end of L is connected to D

In some embodiments, D is

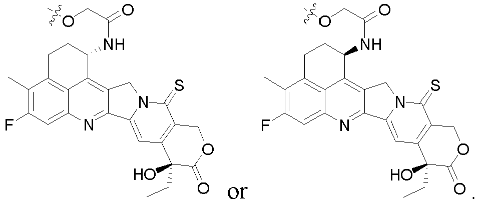

In some embodiments, D is

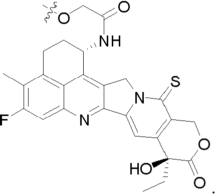

In some embodiments, D is

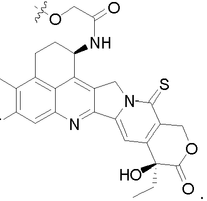

In some embodiments, L-D as a whole is selected from the group consisting of: 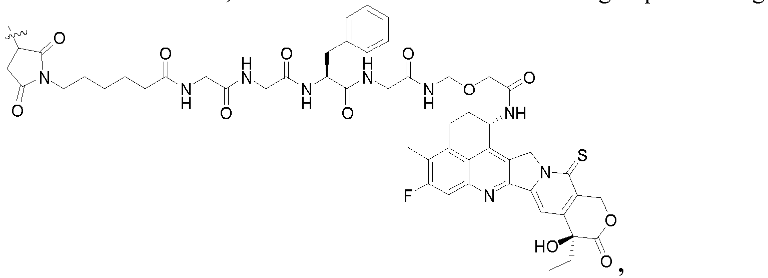 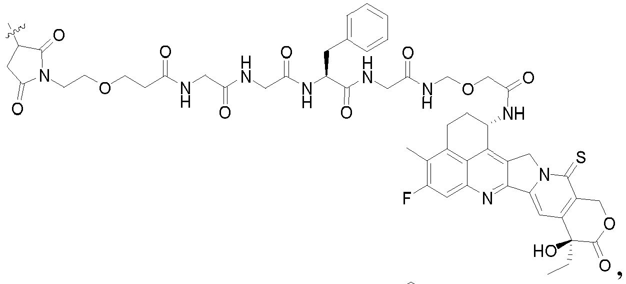 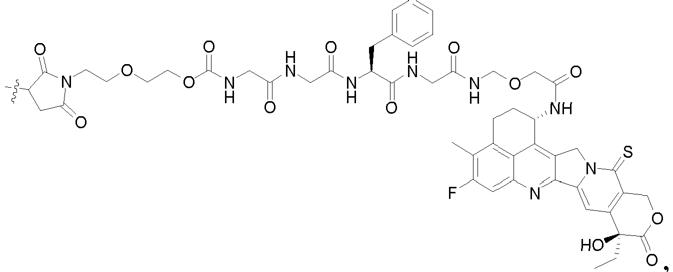 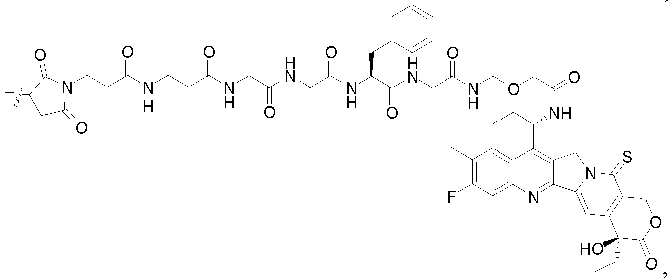 and

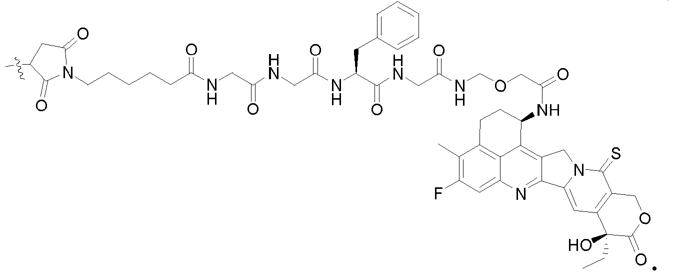

In some embodiments, the antibody-drug conjugate represented by formula I is selected from the group consisting of:

| No. | Structural formula |
|---|---|
| **MH-ADC1** | 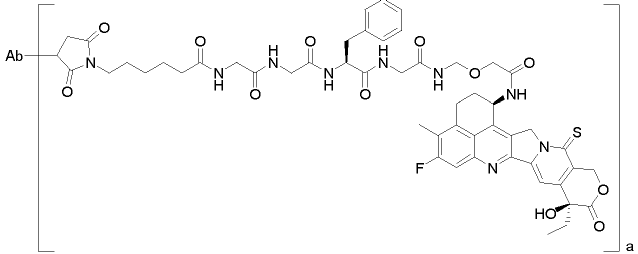 |
| **MH-ADC2** | 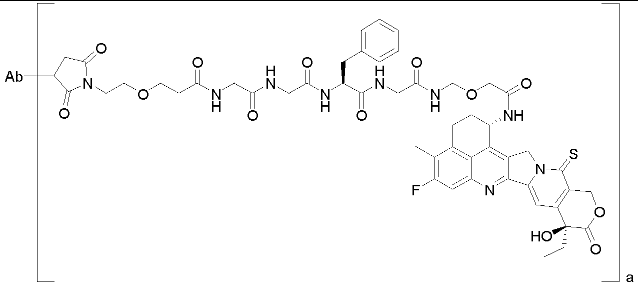 |
| **MH-ADC3** | 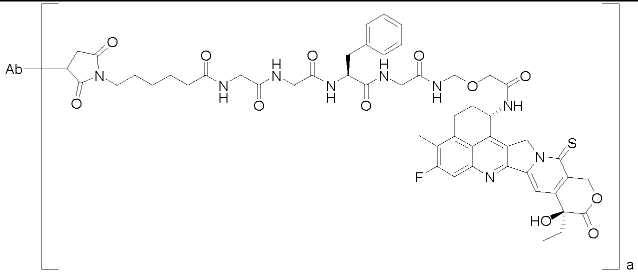 |
| **MH-ADC4** | 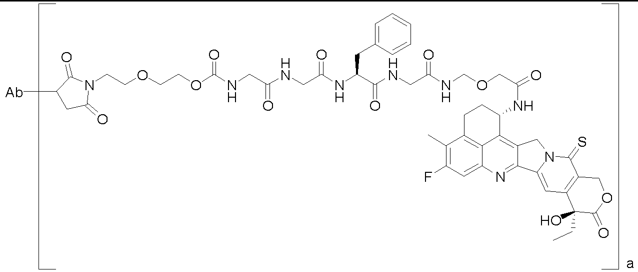 |
| **MH-ADC5** | 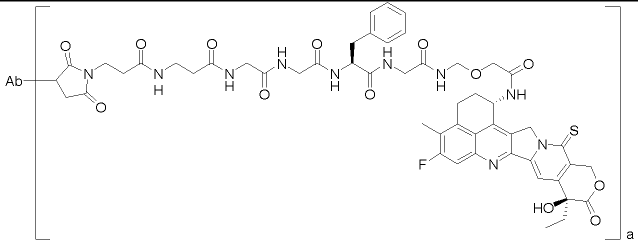 |

wherein Ab and a are defined as described in the present disclosure.

In some embodiments, each a is independently any number between 1 and 8 (such as about 1, about 1.5, about 2, about 2.5, about 3, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, about 7, about 7.1, about 7.2, about 7.3, about 7.4, about 7.5, about 7.6, about 7.7, about 7.8, about 7.9 or about 8, or such as any number between 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5 or 7.5-8).

In some embodiments, each a is independently any number between 3 and 8.

In some embodiments, each a is independently any number between 3 and 6.

In some embodiments, each a is independently any number between 3.5 and 4.5 (such as about 3.5, about 3.6, about 3.7, about 3.8, about 3.9, about 4.0, about 4.1, about 4.2, about 4.3, about 4.4, or about 4.5), for example, each a is independently about 3.9 or about 4.0.

In some embodiments, Ab is an anti-B7-H3 antibody or an antigen-binding fragment thereof.

In some embodiments, the anti-B7-H3 antibody or the antigen-binding fragment thereof comprises:
(a) three heavy chain variable region (VH) complementarity determining regions (CDRs) as described below:
   (i) a VH CDR1, having a CDR1 sequence contained in a VH as shown in SEQ ID NO: 8, or a sequence having one or several amino acids substituted, deleted, or added (e.g., substitution, deletion, or addition of one or two amino acids) as compared to the CDR1 sequence contained in the VH;
   (ii) a VH CDR2, having a CDR2 sequence contained in a VH as shown in SEQ ID NO: 8, or a sequence having one or several amino acids substituted, deleted, or added (e.g., substitution, deletion, or addition of one or two amino acids) as compared to the CDR2 sequence contained in the VH; and
   (iii) a VH CDR3, having a CDR3 sequence contained in a VH as shown in SEQ ID NO: 8, or a sequence having one or several amino acids substituted, deleted, or added (e.g., substitution, deletion, or addition of one or two amino acids) as compared to the CDR3 sequence contained in the VH;
   and/or
(b) three light chain variable region (VL) CDRs as described below:
   (iv) a VL CDR1, having a CDR1 sequence contained in a VL as shown in SEQ ID NO: 12, or a sequence having one or several amino acids substituted, deleted, or added (e.g., substitution, deletion, or addition of one or two amino acids) as compared to the CDR1 sequence contained in the VL;
   (v) a VL CDR2, having a CDR2 sequence contained in a VL as shown in SEQ ID NO: 12, or a sequence having one or several amino acids substituted, deleted, or added (e.g., substitution, deletion, or addition of one or two amino acids) as compared to the CDR2 sequence contained in the VL; and
   (vi) a VL CDR3, having a CDR3 sequence contained in a VL as shown in SEQ ID NO: 12, or a sequence having one or several amino acids substituted, deleted, or added (e.g., substitution, deletion, or addition of one or two amino acids) as compared to the of CDR3 sequence contained in the VL.

In some embodiments, the substitution (or substituted) described in any one of (i) - (vi) is a conservative substitution.

In some embodiments, the CDR1, CDR2, and CDR3 contained in the heavy chain variable region (VH), and/or the CDR1, CDR2, and CDR3 contained in the light chain variable region (VL) are defined by Kabat, Chothia, or IMGT numbering systems.

In some embodiments, the amino acid sequences of heavy chain variable regions CDR1, CDR2, and CDR3 of the anti-B7-H3 antibody or the antigen-binding fragment thereof are shown in SEQ ID NO:5, SEQ ID NO:6, and SEQ ID NO:7, respectively; and the amino acid sequences of light chain variable regions CDR1, CDR2, and CDR3 of the anti-B7-H3 antibody or the antigen-binding fragment thereof are shown in SEQ ID NO:9, SEQ ID NO:10, and SEQ ID NO:11, respectively. In some embodiments, CDRs in the present disclosure are defined by Kabat method.

In some embodiments, the amino acid sequence of heavy chain variable region of the anti-B7-H3 antibody or the antigen-binding fragment thereof is shown in SEQ ID NO:8, and the amino acid sequence of light chain variable region of the anti-B7-H3 antibody or the antigen-binding fragment thereof is shown in SEQ ID NO: 12.

In some embodiments, the amino acid sequence of heavy chain of the anti-B7-H3 antibody is shown in SEQ ID NO:1 and the amino acid sequence of light chain of the anti-B7-H3 antibody is shown in SEQ ID NO:2.

In a second aspect of the present disclosure, the present disclosure provides a pharmaceutical composition comprising the antibody-drug conjugate described above, a stereoisomer thereof, a prodrug thereof, a pharmaceutically acceptable salt thereof, or a pharmaceutically acceptable solvate thereof, and optionally one or more pharmaceutical excipients. In some embodiments, the antibody-drug conjugate, a stereoisomer thereof, a prodrug thereof, a pharmaceutically acceptable salt thereof, or a pharmaceutically acceptable solvate thereof is present in an effective amount.

In a third aspect of the present disclosure, the present disclosure provides a use of the antibody-drug conjugate described above, a stereoisomer thereof, a prodrug thereof, a pharmaceutically acceptable salt thereof, or a pharmaceutically acceptable solvate thereof, or the pharmaceutical composition described above in the manufacture of a medicament for the treatment and/or prevention of a disease, wherein the disease is a disease associated with abnormal B7-H3 expression or function.

In some embodiments, the disease is a cancer or an autoimmune disease.

In some embodiments, the disease is selected from the group consisting of lung cancer (e.g., undifferentiated carcinoma of lung, small cell lung cancer, non-small cell lung cancer), esophageal cancer, gastric cancer, liver cancer, melanoma, prostate cancer, ovarian cancer, endometrial cancer, cervical cancer, breast cancer, head and neck cancer, colorectal cancer, pancreatic cancer, thyroid cancer, sarcoma, cholangiocarcinoma, glioblastoma, and neuroblastoma.

In a fourth aspect of the present disclosure, the present disclosure provides the antibody-drug conjugate described above, a stereoisomer thereof, a prodrug thereof, a pharmaceutically acceptable salt thereof, or a pharmaceutically acceptable solvate thereof, or the pharmaceutical composition described above, for use in the treatment and/or prevention of a disease, wherein the disease is a disease associated with abnormal B7-H3 expression or function.

In some embodiments, the disease is a cancer or an autoimmune disease.

In some embodiments, the disease is selected from the group consisting of lung cancer (e.g., undifferentiated carcinoma of lung, small cell lung cancer, non-small cell lung cancer), esophageal cancer, gastric cancer, liver cancer, melanoma, prostate cancer, ovarian cancer, endometrial cancer, cervical cancer, breast cancer, head and neck cancer, colorectal cancer, pancreatic cancer, thyroid cancer, sarcoma, cholangiocarcinoma, glioblastoma, and neuroblastoma.

In a fifth aspect of the present disclosure, the present disclosure provides a method for treating and/or preventing a disease, comprising administering to an individual in need thereof an effective amount of the antibody-drug conjugate described above, a stereoisomer thereof, a prodrug thereof, a pharmaceutically acceptable salt thereof, or a pharmaceutically acceptable solvate thereof, or the pharmaceutical composition described above, wherein the disease is a disease associated with abnormal B7-H3 expression or function. In some embodiments, the disease is a cancer or an autoimmune disease.

In some embodiments, the disease is selected from the group consisting of lung cancer (e.g., undifferentiated carcinoma of lung, small cell lung cancer, non-small cell lung cancer), esophageal cancer, gastric cancer, liver cancer, melanoma, prostate cancer, ovarian cancer, endometrial cancer, cervical cancer, breast cancer, head and neck cancer, colorectal cancer, pancreatic cancer, thyroid cancer, sarcoma, cholangiocarcinoma, glioblastoma, and neuroblastoma.

In a sixth aspect of the present disclosure, the present disclosure provides a composition for treating and/or preventing a disease, comprising the antibody-drug conjugate described above, a stereoisomer thereof, a prodrug thereof, a pharmaceutically acceptable salt, thereof or a pharmaceutically acceptable solvate thereof. In some embodiments, the disease is a disease associated with abnormal B7-H3 expression or function. In some embodiments, the disease is a cancer or an autoimmune disease. In some embodiments, the disease is selected from the group consisting of lung cancer (e.g., undifferentiated carcinoma of lung, small cell lung cancer, non-small cell lung cancer), esophageal cancer, gastric cancer, liver cancer, melanoma, prostate cancer, ovarian cancer, endometrial cancer, cervical cancer, breast cancer, head and neck cancer, colorectal cancer, pancreatic cancer, thyroid cancer, sarcoma, cholangiocarcinoma, glioblastoma, and neuroblastoma.

In a seventh aspect of the present disclosure, the present disclosure provides a medication for treating and/or preventing a disease, comprising the antibody-drug conjugate described above, a stereoisomer thereof, a prodrug thereof, a pharmaceutically acceptable salt thereof, or a pharmaceutically acceptable solvate thereof as the active ingredient. In some embodiments, the disease is a disease associated with abnormal B7-H3 expression or function. In some embodiments, the disease is a cancer or an autoimmune disease. In some embodiments, the disease is selected from the group consisting of lung cancer (e.g., undifferentiated carcinoma of lung, small cell lung cancer, non-small cell lung cancer), esophageal cancer, gastric cancer, liver cancer, melanoma, prostate cancer, ovarian cancer, endometrial cancer, cervical cancer, breast cancer, head and neck cancer, colorectal cancer, pancreatic cancer, thyroid cancer, sarcoma, cholangiocarcinoma, glioblastoma, and neuroblastoma.

### Definition of Terms

In the present disclosure, unless otherwise specified, the scientific and technical terms used herein have the meanings commonly understood by those skilled in the art. Also, for a better understanding of the present disclosure, definitions and explanations of relevant terms are provided below.

In the present disclosure, the term "pharmaceutically acceptable salt" means (i) salts formed by acidic functional groups present in the compounds provided by the present disclosure with appropriate inorganic or organic cations (bases) including, but not limited to, alkali metal salts such as sodium, potassium, or lithium salts, etc.; alkaline earth metal salts such as calcium, or magnesium salts, etc.; other metal salts such as aluminum, iron, zinc, copper, nickel, or cobalt salts, etc.; inorganic alkali salts such as ammonium salts; organic alkali salts such as tertiary octylamine salt, dibenzylamine salt, morpholine salt, glucosamine salt, phenylglycine alkyl ester salt, ethylenediamine salt, N-methyl glucosamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenylethylamine salt, piperazine salt, tetramethylamine salt, tris(hydroxymethyl) aminomethane salt; and, **(ii)** salts formed by the basic functional groups present in the compounds provided by the present disclosure with appropriate inorganic or organic anions (acids) including, but not limited to, hydrohalide salts, such as hydrofluoride, hydrochloride, hydrobromide, hydroiodide, etc.; inorganic acid salts, such as nitrate, perchlorate, sulfate, phosphate, etc.; lower alkylsulfonates, such as methanesulfonate, trifluoromethanesulfonate, ethanesulfonate etc.; aryl sulfonates, such as benzenesulfonate, *p*-benzenesulfonate, etc.; organic acid salts, such as acetate, malate, fumarate, succinate, citrate, tartrate, oxalate, maleate, etc.; amino acid salts, such as glycinate, trimethylglycinate, argininate, ornithinate, glutamate, aspartate, etc.

Pharmaceutically acceptable salts can be obtained using standard procedures well-known in the field, such as by reacting a sufficient amount of alkaline compounds with suitable acids that provide pharmaceutically acceptable anions, or by reacting a sufficient amount of acidic compounds with suitable alkalis that provide pharmaceutically acceptable cations.

In the present disclosure, the term "prodrug" refers to a derivative that can be hydrolyzed, oxidized, or otherwise reacted under biological conditions (*in vitro* or *in vivo*) to provide a compound of the present disclosure. The prodrugs provide active compounds only under biological conditions as a result of the reaction, or they are inactive or have lower activity in their unreacted form. Prodrugs can generally be prepared using well-known methods, such as those described in Burger's Medicinal Chemistry and Drug Discovery (1995) 172-178, 949-982 (edited by Manfred E. Wolff, 5th ed.).

Stereoisomers of the compounds described in the present disclosure, when specifically designated by chemical name as *(R)-* or *(S)*-isomers, are to be understood as having major configuration of *(R)*-isomer or *(S)*-isomer, respectively. Any asymmetric carbon atom could be present in the *(R)-, (S)-* or (*R,S*)- configuration, preferably present in the *(R)-* or *(S)-* configuration.

In the present disclosure, "solvate" or "solvent compound" could be used interchangeably to refer to a compound that exists in a combination with a solvent molecule. The combination may include a stoichiometric amount of a solvent, such as one molecule of water for forming a monohydrate or two molecules of water for forming a dihydrate, or any amount of water; or such as methanol or ethanol for forming an "alcoholate", which may also be stoichiometric or non-stoichiometric. As used herein, the term "solvate" refers to a solid form, i.e., a compound in solution of a solvent, which although may be solvated, is not a solvate as the term is used herein.

In the present disclosure, the pharmaceutical excipients refer to excipients and additives used in drug manufacturing and formulating, and are substances, in addition to active ingredients, which have been reasonably evaluated in terms of safety and are contained in pharmaceutical preparations. In addition to being used as excipients, acting as carriers and improving stability, which also have important functions such as solubilizing, co-solubilizing, and sustained and controlled releasing, pharmaceutical excipients are important ingredients that may affect the quality, safety and efficacy of drugs. According to sources of pharmaceutical excipients, they can be classified as natural substances, semi-synthetic substances and fully synthetic substances. According to effects and uses of pharmaceutical excipients, they can be classified into: solvents, propellants, solubilizers, co-solvents, emulsifiers, colorants, adhesives, disintegrants, filling agents, lubricants, wetting agents, osmotic pressure regulators, stabilizers, glidants, flavoring agents, preservatives, suspending agents, coating materials, aromatizers, anti-adhesion agents, antioxidants, chelating agents, penetration enhancers, pH regulators, buffers, plasticizers, surfactants, foaming agent, defoamer, thickeners, inclusion agents, humectants, absorbents, diluents, flocculants and deflocculants, filter aids, release retardants, etc. According to administration routes, pharmaceutical excipients can be classified into oral administration, injection, mucosal, transdermal or topical administration, nasal or oral inhalation and ocular administration, etc. The same pharmaceutical excipient can be used in pharmaceutical formulations with different administration routes and have different effects and uses.

In the present disclosure, the pharmaceutical compositions, depending on administration route, can be formulated into various suitable dosage forms, for example, tablets, capsules, granules, oral solutions, oral suspensions, oral emulsions, powders, tinctures, syrups, injections, suppositories, ointments, creams, pastes, ophthalmic preparations, pills, implants, aerosols, powders, sprays and the like. Among them, the pharmaceutical compositions or suitable dosage forms may comprise 0.01 mg to 1,000 mg, suitably 0.1 mg to 800 mg, preferably 0.5-500 mg, further preferably 0.5 to 350 mg, and particularly preferably 1 to 250 mg of the antibody-drug conjugate of the present disclosure, a stereoisomer thereof, a prodrug thereof, a pharmaceutically acceptable salt thereof, or a pharmaceutically acceptable solvate thereof.

In the present disclosure, the term "treatment" generally refers to obtain a desired pharmacological and/or physiological effect. The effect could be prophylactic based on completely or partially preventing a disease or symptom; and/or therapeutic based on partially or completely stabilizing or curing a disease and/or side effects caused by the disease. As used herein, "treatment" encompasses any treatment of a disease in a patient, including: (a) preventing diseases or symptoms from occurring in a patient who is susceptible to the disease or symptom but has not yet been diagnosed with; (b) suppressing symptoms of a disease, i.e., stopping its progression; or (c) alleviating symptoms of a disease, i.e., causing the disease or symptoms to degenerate.

In the present disclosure, the term "individual" includes human or non-human animal. Exemplary human individual includes human individuals (referred to as patients) suffering from a disease (e.g., a disease described herein) or normal individuals. The term "non-human animal" in the present disclosure includes all vertebrates, such as non-mammals (e.g., birds, amphibians, reptiles) and mammals, such as non-human primates, livestock, and/or domesticated animals (e.g., sheep, dogs, cats, cows, pigs, etc.).

In the present disclosure, the term "effective amount" refers to an amount of a compound that, when administered, provides some alleviation of one or more symptoms of the condition being treated.

In the present disclosure, the term "antibody" is to be interpreted in its broadest sense and includes intact monoclonal antibodies, polyclonal antibodies, and multi-specific antibodies (e.g., bispecific antibodies) formed from at least two intact antibodies as long as they have the desired biological activity. In the present disclosure, the terms "antibody" and "immunoglobulin" could be used interchangeably.

In the present disclosure, the term "monoclonal antibody" refers to an antibody derived from a group of substantially uniform antibodies, i.e., the antibodies composing the group are identical, except for a small number of natural mutations that may be present. A monoclonal antibody has high specificity for one determinant (epitope) of an antigen, whereas a polyclonal antibody in contrast contains different antibodies for different determinants (epitopes). In addition to specificity, monoclonal antibodies have the advantage that they can be synthesized without contamination by other antibodies. The modifier "monoclonal" herein indicates that the antibody is characterized as being derived from a substantially homogeneous group of antibodies and should not be understood to indicate that it is produced by a special method.

In some embodiments of the present disclosure, the monoclonal antibody further comprises, in particular, a chimeric antibody, i.e., a portion of the heavy and/or light chain is identical or homologous to a certain type, class, or subclass of antibody, and the remainder of the heavy and/or light chain is identical or homologous to another type, class, or subclass of antibody as long as they have the desired biological activity (see, e.g., US 4,816,567; and Morrison et al., 1984, PNAS, 81: 6851-6855). Chimeric antibodies that can be used in the present disclosure include primatized antibodies comprising variable region antigen-binding sequences from non-human primates (e.g., ancient monkeys, orangutans, etc.) and human constant region sequences.

Monoclonal antibodies used in the present disclosure can be produced by many methods. For example, monoclonal antibodies used in the present disclosure can be obtained by hybridoma methods using cells from many species (including cells from mice, hamsters, rats, and humans) (see e.g., Kohler et al., 1975, Nature, 256: 495), or produced by recombinant DNA techniques (see e.g., US 4,816,567), or isolated from phage antibody libraries (see for example Clackson et al., 1991, Nature, 352: 624-628; and Marks et al., 1991, Journal of Molecular Biology, 222: 581-597).

In the present disclosure, unless otherwise expressly specified, the descriptors "each ... independently selected from" and "...each independently selected from" used throughout herein could be used interchangeable and are to be interpreted in broad sense, meaning that the specific options expressed by the same or different symbols in different groups do not affect each other, or the specific options expressed by the same or different symbols in the same groups do not affect each other.

In the present disclosure, "n1 is an integer selected from 1 to 5" refers to n1 being selected from 1, 2, 3, 4, and 5. Other similar definitions can be understood with reference to the foregoing.

In the present disclosure, the term "bond" indicates that the groups on both sides are directly connected, for example, L¹ is 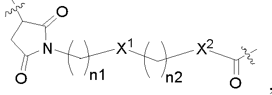 , and if X¹ is a bond, L¹ is 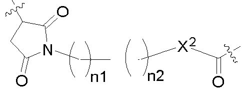 . Other similar definitions can be understood with reference to the foregoing.

In the present disclosure, "L¹ is 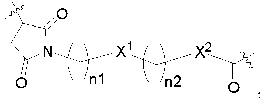 and the carbonyl carbon end of L¹ is connected to L², and the alkyl carbon end of L¹ is connected to Ab", wherein, the "carbonyl carbon end" or "alkyl carbon end" of L¹ is used to describe the connection relationship between L¹ and other groups. Specifically, the "carbonyl carbon end" of L¹ refers to the carbonyl carbon end located at the connection site on the right end of L¹, and the "alkyl carbon end" of L¹ refers to the alkyl carbon end located at the connection site on the left end of L¹. Other similar definitions can be understood with reference to the foregoing.

In the present disclosure, the amino end of L³ is connected to L² and the carbonyl end of L³ is connected to L⁴, but if L² is a bond, it can be understood by those skilled in the art that the amino end of L³ will be connected to L¹ accordingly, and the carbonyl end will remain to be connected to L⁴. Other similar situations can be understood with reference to the foregoing.

In the present disclosure, the term "amino acid residue" refers to a remainder incomplete amino acid structure that is formed after the amino group of an amino acid loses a hydrogen and the carboxyl group of the amino acid loses a hydroxyl group.

In the present disclosure, L³ is an amino acid residue or a peptide residue consisting of 2 to 10 amino acid residues, wherein the types of 2 to 10 amino acids may be the same or different. For example, if L³ is a peptide residue consisting of 4 amino acid residues and the amino acid is selected from the group consisting of glycine and phenylalanine, L³ could be a peptide residue such as glycine-glycine-phenylalanine-glycine (Gly-Gly-Phe-Gly), and specifically could be

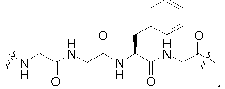

In each part of the present disclosure, the substituents of compounds of the present disclosure are disclosed according to the type or range of groups. It should be particularly indicated that, every independent subordinated combination of the types and scope of these groups are encompassed by the present disclosure. For example, the term "C1-C6 alkyl" specifically refers to independently disclosed methyl, ethyl, C3 alkyl, C4 alkyl, C5 alkyl, and C6 alkyl.

In the present disclosure, the term "C1-C6 alkyl" refers to straight or branched alkyl groups containing 1-6 carbon atoms, including, for example, "C1-C3 alkyl" or "C1-C4 alkyl", methyl, ethyl, etc., specific examples include, but are not limited to: methyl, ethyl, n-propyl, isopropyl, n-butyl, isobutyl, sec-butyl, tert-butyl, pentyl, hexyl.

In the present disclosure, the term "C1-C4 alkyl" refers to straight or branched alkyl groups containing 1-4 carbon atoms, including, for example, "C1-C3 alkyl", methyl, ethyl, etc., specific examples include but are not limited to: methyl, ethyl, n-propyl, isopropyl, n-butyl, isobutyl, sec-butyl, tert butyl.

Halogen refers to fluorine, chlorine, bromine or iodine.

In the present disclosure, the term "halogenated C1-C6 alkyl" refers to a group obtained by substituting one or more hydrogen atoms in any of the aforementioned alkyl groups (e.g., C1-C6 alkyl, C1-C4 alkyl, C1-C3 alkyl, etc.) with halogens (preferably fluorine, chlorine), e.g., monofluoromethyl, difluoroethyl, trifluoromethyl, etc.

In the present disclosure, the term "deuterated C1-C6 alkyl" refers to a group obtained by substituting one or more hydrogen atoms in any of the aforementioned alkyl groups (e.g. C1-C6 alkyl, C1-C4 alkyl, C1-C3 alkyl, etc.) with deuterium atoms, e.g. monodeuteromethyl, dideuteromethyl, trideuteromethyl, etc.

Those skilled in the art can understand that in the antibody-drug conjugate disclosed in the present disclosure, the antibody is connected to the linker-payload through -S-, and -S- is not an additional external sulfhydryl group, but the sulfhydryl group generated by antibody itself after being reduced and the disulfide bond being opened.

In the present disclosure, the drug antibody ratio (DAR) refers to the number of drug molecules conjugated to the antibody (for example, a in formula I). The number of drug molecules contained in the antibody-drug conjugates described herein could be integral or decimal. Whether an integer or a decimal, it refers to an average number of drug molecules conjugated per antibody. the term "a is any number between 1 and 10" means that a can be any integer selected from between 1 and 10 (including endpoints 1 and 10) or any decimal selected from between 1 and 10, such as 3.9 or 4.0. At the same time, it can be understood by those skilled in the art that even if the same preparation method is used, the DAR values of the antibody-drug conjugates obtained from different batches of preparation may not necessarily be exactly the same, for example, they may fluctuate up and down within 0.5.

In the present disclosure, the term "about" is understood to be within +/-10%, +/-9%, +/-8%, +/-7%, +/-6%, +/-5%, +/-4%, +/-3%, +/-2%, +/-1%, +/-0.5%, +/-0.4%, +/-0.3%, +/-0.2%, +/-0.1 % of the stated value. All values provided herein are modified by the term "about" unless otherwise apparent from the context.

In the present disclosure, the term "conservative substitution" refers to an amino acid substitution that does not adversely affect or alter the necessary properties of the protein/polypeptide comprising the amino acid sequence. For example, conservative substitution can be introduced by standard techniques known in the art such as site-directed mutagenesis and PCR-mediated mutagenesis. Conservative amino acid substitution includes the substitution of amino acid residues with amino acid residues having similar side chains, such as substitution with a residue that is physically or functionally similar to the corresponding amino acid residue (e.g., having a similar size, shape, charge, chemical properties including the ability to form covalent or hydrogen bonds, etc.). Families of amino acid residues having similar side chains have been defined in the art. These families include amino acid having basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), amino acids with beta-branched side chains (e.g., threonine, valine, isoleucine), and aromatic side chains (e.g., tyrosine, phenylalanine, tryptophan, histidine). Thus, it is preferred to replace the corresponding amino acid residue with another amino acid residue from the same side chain family. Methods for identifying conservative amino acid substitution are well known in the art (see, e.g., Brummell et al. Biochem. 32: 1180-1187 (1993); Kobayashi et al. Protein Eng. 12(10): 879-884 (1999); and Burks et al. Proc. Natl Acad. Sci. USA 94: 412-417 (1997), which are incorporated herein by reference).

### Beneficial Effects

1. The ADCs of the present disclosure show excellent inhibitory activities against multiple tumor cell lines with different target expression levels.
2. The ADCs of the present disclosure show strong *in vivo* proliferation-inhibition effects in subcutaneous xenograft tumor models in nude mice with human-derived tumor cells.

### Description of the Drawings

The drawings described herein are used to provide a further understanding of the present disclosure and constitute a part of the present application, and the exemplary embodiments of the present disclosure and their descriptions are used to explain the present disclosure and do not constitute an undue limitation of the present disclosure. In the drawings:
Figure 1 shows tumor growth curves of each administration group in human lung cancer NCI-H1975 subcutaneous xenograft tumor model in nude mice;
Figure 2 shows tumor growth curves of each administration group in human lung cancer Calu-6 subcutaneous xenograft tumor model in nude mice;
Figure 3 shows tumor growth curves of each administration group in human colorectal cancer HCT116 subcutaneous xenograft tumor model in nude mice.

### Detailed Description of the Embodiments

The present disclosure is further illustrated below by the description of specific examples, but this is not a limitation on the present disclosure. Various modifications or improvements may be made by those skilled in the art in accordance with the teachings of the present disclosure without departing from the basic idea and scope of the present disclosure. The reagents or instruments used without indication of the manufacturer are conventional products that are commercially available.

In the following embodiments, the abbreviations of some of the drugs and their structural formula correspond as follows:

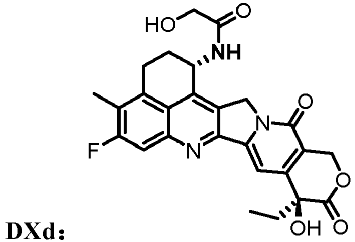

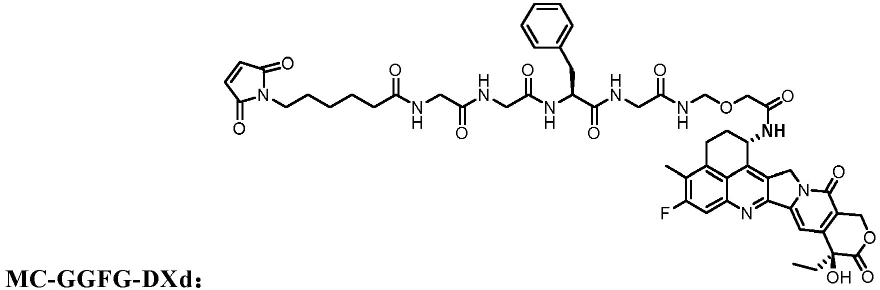

Wherein, MC-GGFG-DXd can be prepared and obtained with reference to Example 58 of CN104755494A.

In the following examples, the antibodies used are known antibodies, and the sequences of the antibodies are as shown below:
The CDRs (shown underlined) are defined below using Kabat method.

Antibody P7-C05-H4L3 sequence (see WO2021244590A1):
>Heavy chain

EVQLVESGGGLVQPGGSLRLSCAASGFTFSSYAMSWVRQAPGKGLELVAAINS
NGGSTYYADSVKGRFTISRDNAKNTLYLQMSSLRAEDTAVYYCTRNEEFRRGLAYW
GQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GK（SEQ ID NO:1）

Wherein, from left to right, the part without gray shadow is the heavy chain variable region sequence (SEQ ID NO: 8), and the part with gray shadow is the heavy chain constant region sequence. In the heavy chain variable region sequence, the underlined parts from left to right are the heavy chain CDR1 sequence (SEQ ID NO: 5), the heavy chain CDR2 sequence (SEQ ID NO: 6), and the heavy chain CDR3 sequence (SEQ ID NO: 7), successively.
>Light chain

DIQMTQSPSSLSASVGDRVTITCRASENIYSNLAWYQQKPGKAPKLLIYAASNL
QSGVPSRFSGSGSGTDYTLTISSLQPEDFATYYCQHFWGTPPWTFGQGTKLEIKRTV
AAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC （ SEQ ID
NO:2）

Wherein, from left to right, the part without gray shadow is the light chain variable region sequence (SEQ ID NO:12), and the part with gray shadow is the light chain constant region sequence. In the light chain variable region sequence, the underlined parts from left to right are the light chain CDR1 sequence (SEQ ID NO:9), the light chain CDR2 sequence (SEQ ID NO: 10), and the light chain CDR3 sequence (SEQ ID NO:11), successively.

The sequence of control ADC2 antibody (Ifinatamab) (see Reference Example 1, i.e., antibody M30-H1-L4, in CN 104755494 A):
>Heavy chain

QVQLVQSGAEVKKPGSSVKVSCKASGYTFTNYVMHWVRQAPGQGLEWMGYI
NPYNDDVKYNEKFKGRVTITADESTSTAYMELSSLRSEDTAVYYCARWGYYGSPLY
YFDYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKRVE
PKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYTQK
SLSLSPGK (SEQ ID NO:3)

Wherein, from left to right, the part without gray shadow is the heavy chain variable region sequence (SEQ ID NO: 16), and the part with gray shadow is the heavy chain constant region sequence. In the heavy chain variable region sequence, the underlined parts from left to right are the heavy chain CDR1 sequence (SEQ ID NO: 13), the heavy chain CDR2 sequence (SEQ ID NO: 14), and the heavy chain CDR3 sequence (SEQ ID NO: 15), successively.
>Light chain

EIVLTQSPATLSLSPGERATLSCRASSRLIYMHWYQQKPGQAPRPLIYATSNLAS
GIPARFSGSGSGTDFTLTISSLEPEDFAVYYCQQWNSNPPTFGQGTKVEIKRTVAAPS

VFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC（SEQ ID NO:4）

Wherein, from left to right, the part without gray shadow is the light chain variable region sequence (SEQ ID NO:20), and the part with gray shadow is the light chain constant region sequence. In the light chain variable region sequence, the underlined parts from left to right are the light chain CDR1 sequence (SEQ ID NO:17), the light chain CDR2 sequence (SEQ ID NO:18), and the light chain CDR3 sequence (SEQ ID NO:19), successively.

The present disclosure is further explained and illustrated by reference to the following specific examples. Unless otherwise specified, the reaction materials are commercially available.

### Example 1 preparation of linker-payloads

### 1.1 Preparation of compound II-12-a

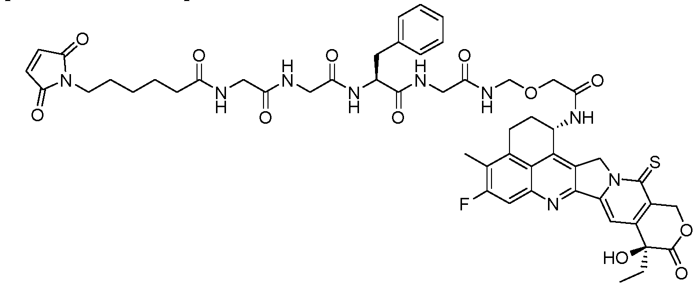

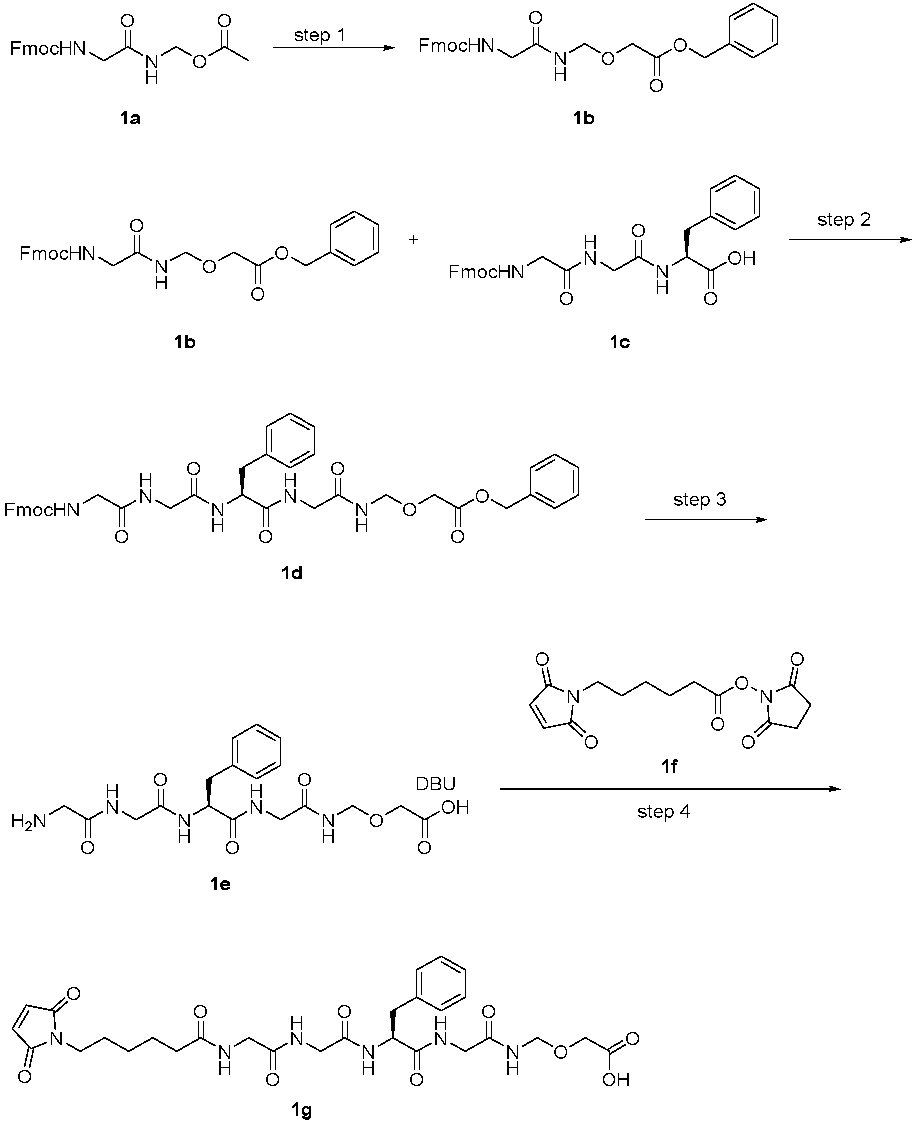

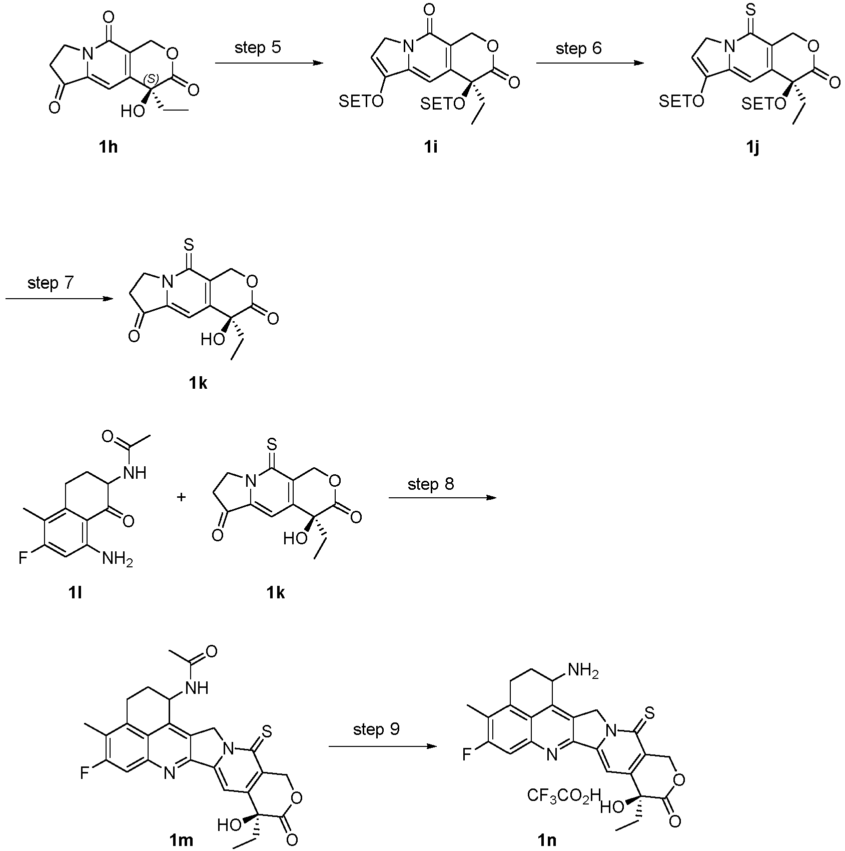

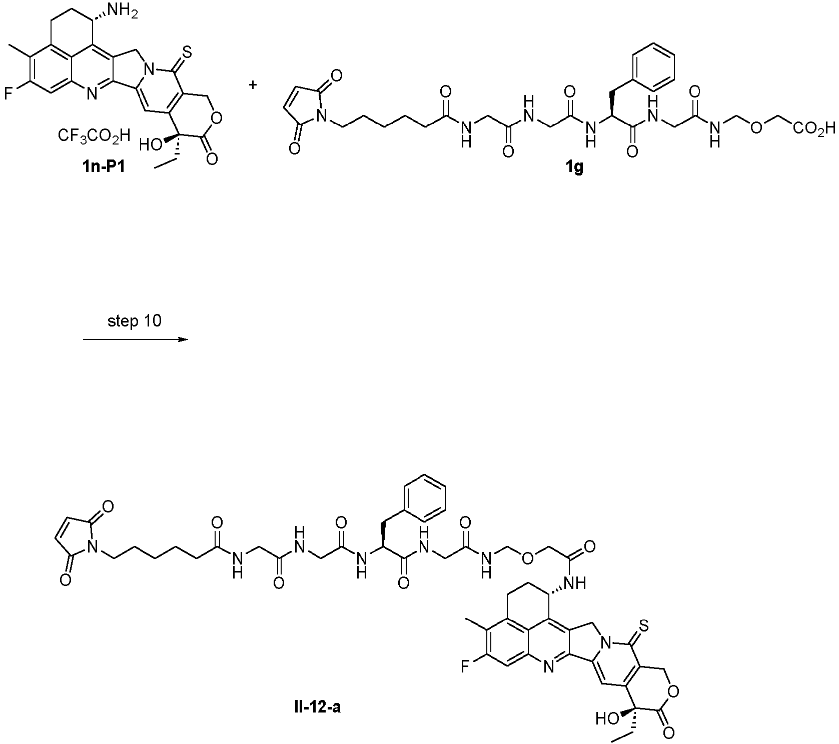

### Step 1

Compound **1a** (50.00 g, 135.90 mmol) and benzyl glycolate (45.00 g, 271.70 mmol) were added to a three-neck flask (1 L) successively. Then the atmosphere was replaced with N₂, and anhydrous tetrahydrofuran (500 mL) was added. The flask was then placed in an ice bath followed by addition of anhydrous *p*-toluenesulfonic acid (2.30 g, 13.40 mmol), and the resultant reaction mixture was stirred for 2 hours under ice bath. Water (1 L) and ethyl acetate (1 L) were added to the reaction mixture, and the layers were separated. The organic phase was washed by aq. sodium bicarbonate, water and aq. sodium chloride successively. The organic phase was dried over anhydrous sodium sulfate and then filtrated. The filtrate was concentrated *in vacuo* and the residue was purified by flash column chromatography on silica gel (methanol: dichloromethane = 0-100%) to afford compound **1b** (27.00 g) with 42% yield.

MS-ESI calc. for [M+Na]⁺ 497, found: 497.

### Step 2

Compound **1b** (17.00 g, 35.86 mmol) was dissolved in *N,N*-dimethylacetamide (170 mL), and to the obtained solution 1,8-Diazabicyclo[5.4.0]undecane-7-ene (2.78 g, 18.29 mmol) was added. The reaction mixture was stirred at room temperature for 1 hour under N₂ atmosphere. Pyridinium *p*-toluenesulfonate (4.59 g, 18.29 mmol), 1-hydroxybenzotriazole (4.59 g, 34.00 mmol), **1c** (16.66 g, 33.18 mmol) and 1-(3-dimethylaminopropyl)-3-ethylcarbodiimide hydrochloride (6.37 g, 33.18 mmol) were added to the above reaction mixture. After addition was completed, the resulting mixture was stirred at room temperature for an additional 2.5 hours. 2-Methyltetrahydrofuran (175 mL) and saturated aq. sodium chloride (260 mL) were added to the reaction mixture, and the organic phase was extracted and separated. The aqueous phase was extracted with 2-methyltetrahydrofuran (100 mL × 2). The obtained organic phases were combined. The combined organic phases were washed with 10% citric acid once (90 mL), aq. sodium bicarbonate three times (175 mL×3) and saturated brine once (100 mL) successively, dried over anhydrous sodium sulfate and then filtrated. The filtrate was concentrated *in vacuo* to approximately 50 mL, and isopropanol (120 mL) was added. The obtained solution was reconcentrated *in vacuo* to approximately 50 mL followed by addition of isopropanol (300 mL). The obtained solution was stirred at 60°C for 1h, and subsequently cooled to 0-5°C, stirred at 0-5°C for 2 h, and filtered. The obtained solid was washed with isopropanol (5°C) to afford compound **1d** (19.00 g) with 78% yield.

### Step 3

Compound **1d** (11.50 g, 15.65 mmol) was dissolved in a mixed solvent of tetrahydrofuran (230 mL) and H₂O (115 mL), and to the obtained solution 1,8-Diazabicyclo[5.4.0]undecane-7-ene (10.70 g, 70.39 mmol) was added. The mixture was stirred at room temperature for 4 hours under N₂ atmosphere. The reaction mixture was extracted with *tert*-butyl methyl ether five times (100 mL × 5). The aqueous phase was concentrated to obtain yellow oily residue followed by dissolvation in isopropanol (70 mL). The obtained solution was slowly added to *tert*-butyl methyl ether (700 mL). A white solid was precipitated, filtered and collected. The obtained white solid was dissolved with ethanol and concentrated *in vacuo* to afford white foamy solid **1e** (9.50 g) with 93% yield.

### Step 4

Compound **1e** (1.00 g, 1.53 mmol) was dissolved in a mixed solvent of acetonitrile (10 mL) and H₂O (20 mL), and to the obtained solution compound **1f** (0.54 g, 1.75 mmol) was added. The mixture was stirred at room temperature for 5 hours under N₂ atmosphere. Water (20 mL) was added to the mixture, and then the pH was adjusted to 3-4 by adding 0.5N HCl_{(aq)}. The obtained mixture was extracted with a mixed solvent of isopropanol/dichloromethane (V:V=4:1, 50 mL) for 4 times. The obtained organic phases were combined. The combined organic phases were dried over anhydrous sodium sulfate and then filtrated. The filtrate was concentrated *in vacuo* to obtain yellow oily residue followed by dissolvation in a mixed solvent of dichloromethane/methanol (v:v = 4:1, 5 mL). The obtained solution was slowly added to *tert*-butyl methyl ether (100 mL). A white solid was precipitated, filtrated and collected to afford compound **1g** (850 mg) with 90% yield.

MS-ESI calc. for [M+Na]⁺ 639, found: 639.

### Step 5

Compound **1h** (12.20 g, 46.39 mmol) was added to a three-neck flask (500 mL), and the atmosphere was replaced with N₂. The flask was then placed in an ice bath. *N,N-*dimethylformamide (120 mL) was added followed by addition of imidazole (15.77 g, 232.00 mmol) and chlorotriethylsilane (27.97 g, 185.56 mmol) successively. The obtained mixture was stirred for 10 min, then 4-dimethylaminopyridine (5.66 g, 46.39 mmol) was added. The resulting mixture was stirred at 0°C for 3 h. The reaction mixture was diluted with *tert*-butyl methyl ether (1.20 L) followed by addition of water (1.2 L), and the layers were separated. The aqueous phase was extracted with *tert-butyl* methyl ether (1.2 L). The obtained organic phases were combined. The combined organic phases were washed by water once (1.20 L), dried over anhydrous sodium sulfate and then filtrated. The filtrate was concentrated *in vacuo* and the residue was purified by flash column chromatography on silica gel (ethyl acetate: petroleum ether = 0-100%) to afford crude **1i** (16.00 g).

### Step 6

To a three-neck flask (1 L) were added crude **1i** (16.00 g, 32.59 mmol) and Lawesson's reagent (13.18 g, 32.59 mmol) successively followed by addition of anhydrous toluene (600 mL). Then the atmosphere was replaced with N₂, and the reaction mixture was refluxed at 125°C for 5 hours. The reaction mixture was concentrated, and the residue was purified by flash column chromatography on silica gel (ethyl acetate: petroleum ether = 0-100%) to afford crude **1j** (16.00 g).

### Step 7

Crude **1j** (16 g crude) was dissolved in anhydrous tetrahydrofuran (640 mL), and to the obtained solution triethylamine trihydrofluoride (11.93 g, 74.00 mmol) was added dropwise under an ice bath. After addition was completed, the resulting mixture was warmed slowly to room temperature and stirred overnight. The reaction mixture was then diluted with ethyl acetate (1 L) and then the layers were separated. The organic phase was washed by saturated brine (500 mL × 2), dried over anhydrous sodium sulfate and then filtrated. The filtrate was concentrated *in vacuo* and the residue was purified by flash column chromatography on silica gel (ethyl acetate: petroleum ether = 0-100%) to afford compound **1k** (6.38 g) with three-step yield of 49%.

### Step 8

To a three-neck flask (500 mL) were added compound **1l** (3.00 g, 12.00 mmol), **1k** (4.33 g, 15.52 mmol), pyridinium *p*-toluenesulfonate (3.90 g, 15.50 mmol) and o-cresol (15 mL), followed by addition of anhydrous toluene (300 mL). Then the atmosphere was replaced with N₂, and the reaction mixture was stirred at 120 °C under oil bath for 32 hours. The reaction mixture was cooled to room temperature, and the insoluble substance was filtrated and collected. The obtained crude product was triturated with *tert*-butyl methyl ether (60 mL) for 1 hour. The mixture was then filtered, and dried to afford compound **1m** (3.0 g) with 51% yield.

### Step 9

To a single-neck flask (500 mL) was added compound **1m** (2.30 g, 4.67 mmol) followed by addition of 6N HCl_{(aq)} (230 mL). The resulting mixture was refluxed at 110 °C for 12 hours. The reaction mixture was thermally filtered to remove insoluble materials. The filtrate was concentrated *in vacuo* to afford crude product **1n** (a pair of diastereomers). The crude product **1n** was purified by prep-HPLC to afford compounds **1n-P1** (412 mg, isomer with lower polarity in TLC) and **1n-P2** (495 mg, isomer with higher polarity in TLC) with 34% yield.

### Step 10

To a single-neck flask (10 mL) were added compound **1n-P1** (50 mg, 0.09 mmol), **1g** (76 mg, 0.12 mmol), 1-(3-dimethylaminopropyl)-3-ethylcarbodiimide hydrochloride (31 mg, 0.16 mmol) and *N,N*-dimethylformamide (2 mL) successively, followed by dropwise addition of 2,4,6-trimethylpyridine (60 mg, 0.49 mmol). After addition was completed, the resulting mixture was stirred at room temperature for 3 hours. The reaction solution was added dropwise to *tert-butyl* methyl ether (60 mL). A solid was precipitated, filtered and collected. The obtained solid was dissolved in a mixed solvent of dichloromethane/methanol (V:V = 6:1, 30 mL). The obtained solution was concentrated, and the obtained crude was purified by prep-HPLC to afford compound **II-12-a** (33 mg) with 35% yield.

MS-ESI calc. for [M+H]⁺1050, found: 1050.

**¹H NMR (400 MHz, DMSO-*d₆*) δ 8.65 (t, *J =* 6.4 Hz, 1H), 8.60 (d, *J =* 8.8 Hz, 1H), 8.28 (t, *J =* 5.6 Hz, 1H), 8.10 (d, *J =* 8.0 Hz, 1H), 8.06 (t, *J =* 6.0 Hz, 1H), 7.99 (t, *J =* 5.6 Hz, 1H), 7.79 (d, *J =* 10.8 Hz, 1H), 7.78 (s, 1H), 7.28-7.14 (m, 5H), 6.97 (s, 1H), 5.90 (d, *J* = 16.4 Hz, 1H), 5.70-5,60 (m, 1H), 5.51 (s, 1H), 5.46 (d, *J =* 2.8 Hz, 1H), 5.30 (d, *J =* 19.6 Hz, 1H), 4.67 (d*, J =* 6.4 Hz, 1H), 4.51-4.41 (m, 1H), 4.18-4.04 (m, 2H), 3.77-3.73 (m, 1H), 3.73-3.68 (m, 2H), 3.68-3.62 (m, 2H), 3.62-3.58 (m, 1H), 3.58-3.54 (m, 1H), 3.38-3.31 (m, 2H), 3.30-3.20 (m, 1H), 3.20-3.08 (m, 1H), 3.01 (dd, *J* =14.0, 4.8 Hz, 1H), 2.80-2.71 (m, 1H), 2.37 (s, 3H), 2.26-2.15 (m, 2H), 2.08 (t, *J =* 7.6 Hz, 2H), 1.94-1.82 (m, 2H), 1.54-1.37 (m, 4H), 1.25-1.10 (m, 2H), 0.85 (t, *J* =7.6 Hz, 3H).**

### 1.2 Preparation of compound II-1-a

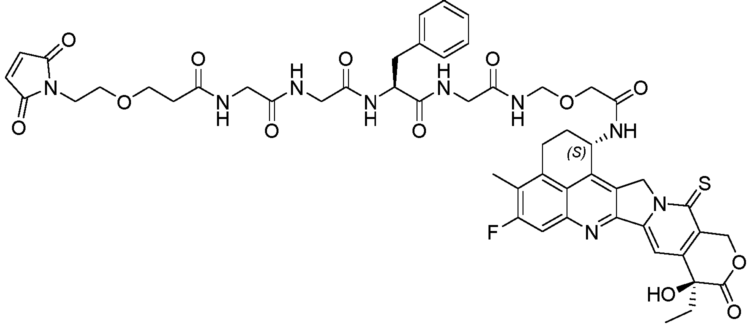

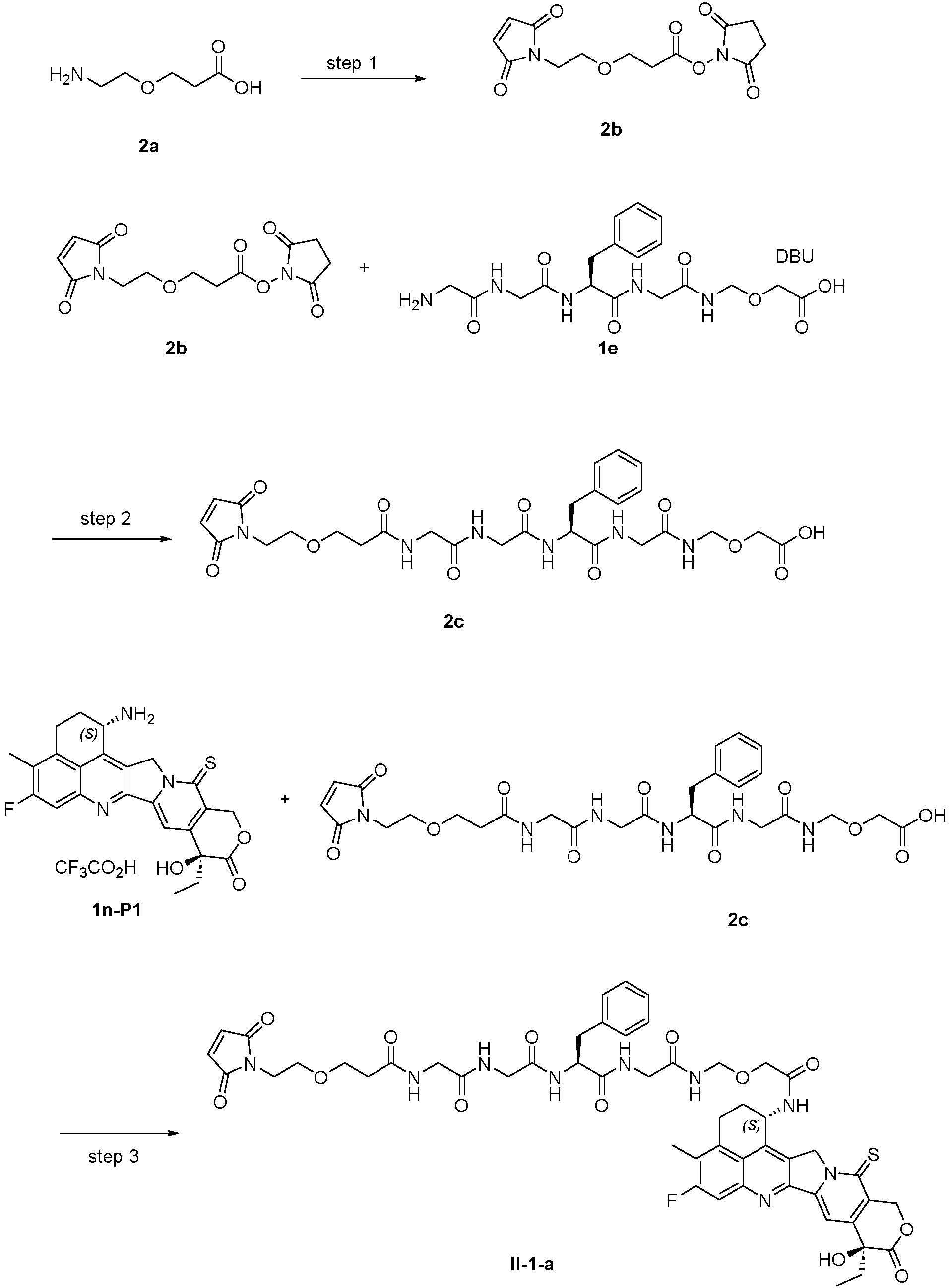

### Step 1

Compound **2a** (1.00 g, 7.52 mmol) was dissolved in *N,N-*dimethylformamide (10 mL), and to the obtained solution maleic anhydride (0.74 g, 7.52 mmol) was added. The reaction mixture was stirred at room temperature for 16 hours under N₂ atmosphere. The resulting reaction solution was recorded as solution A and set aside.

To a three-neck flask (50 mL) were added N-hydroxysuccinimide (3.46 g, 30.08 mmol) and *N,N*-dimethylformamide (10 mL), and the mixture was cooled to 0°C followed by dropwise addition of trifluoroacetic anhydride (6.31 g, 30.08 mmol). After addition was completed, the reaction mixture was stirred for 0.5 h, and then 2,4,6-trimethylpyridine (5.46 g, 45.10 mmol) was added. After addition was completed, the reaction mixture was stirred for an additional 0.5 hour. Then the reaction solution was added dropwise to the above solution A. After addition was completed, the reaction mixture was stirred at room temperature for 16 hours. The reaction mixture was adjusted pH to 2-3 by adding 1N HCl_{(aq)} and extracted with dichloromethane (50 mL). The organic phase was washed with water for three times, dried over anhydrous sodium sulfate and then filtrated. The filtrate was concentrated *in vacuo,* and the residue was purified by flash column chromatography on silica gel (ethyl acetate: petroleum ether = 0-100%) to afford compound **2b** (1.20 g) with 51% yield.

### Step 2

Compound **2b** (0.40 g, 1.29 mmol) was dissolved in a mixed solvent of acetonitrile (8 mL) and water (16 mL), and to the obtained solution compound **1e** (0.54 g, 0.80 mmol) was added. The reaction mixture was stirred at room temperature for 16 hours under N₂ atmosphere. The reaction mixture was concentrated *in vacuo,* and the residue was purified by prep-HPLC to afford compound **2c** (0.30 g) with 61% yield.

### Step 3

To a single-neck flask (10 mL) were added compound **1n-P1** (50 mg, 0.09 mmol), **2c** (76 mg, 0.12 mmol), 1-(3-dimethylaminopropyl)-3-ethylcarbodiimide hydrochloride (31 mg, 0.16 mmol) and *N,N*-dimethylformamide (2 mL) successively, followed by dropwise addition of 2,4,6-trimethylpyridine (60 mg, 0.49 mmol). After addition was completed, the resulting mixture was stirred at room temperature for 3 hours. The reaction solution was added dropwise to *tert*-butyl methyl ether (60 mL). A solid was precipitated, filtered and collected. The obtained solid was dissolved in a mixed solvent of dichloromethane/methanol (V:V = 6:1, 30 mL). The obtained solution was concentrated, and the obtained crude was purified by prep-HPLC to afford compound **II-1-a** (16 mg) with 17% yield.

MS-ESI calc. for [M+H]⁺ 1052, found: 1052.

**¹H NMR (400 MHz, DMSO-*d*₆) δ 8.65 (t, *J =* 6.8 Hz, 1H), 8.61 (d, *J =* 9.2 Hz, 1H), 8.28 (t, *J =* 4.4 Hz, 1H), 8.14-8.06 (m, 2H), 8.06 (t, *J =* 5.6 Hz, 1H), 7.82 (d, *J =* 11.2 Hz, 1H), 7.79 (s, 1H), 7.28-7.13 (m, 5H), 6.99 (s, 2H), 5.92 (d, *J =* 16.8 Hz, 1H), 5.70-5,60 (m, 1H), 5.53 (d, *J =* 8.0 Hz, 1H), 5.48 (d, *J =* 4.8 Hz, 1H), 5.37 (d, *J =* 19.6 Hz, 1H), 4.68 (d, *J =* 6.4 Hz, 1H), 4.51-4.41 (m, 1H), 4.17-4.04 (m, 2H), 3.72-3.68 (m, 2H), 3.68-3.63 (m, 2H), 3.62-3.58 (m, 1H), 3.58-3.54 (m, 3H), 3.54-3.39 (m, 3H), 3.48-3.43 (m, 2H), 3.19-3.08 (m, 1H), 3.06-2.97 (m, 1H), 2.80-2.70 (m, 1H), 2.70-2.65 (m, 1H), 2.39 (s, 3H), 2.35-2.28 (m, 3H), 2.27-2.18 (m, 2H), 1.94-1.82 (m, 2H), 0.85 (t, *J =* 7.2 Hz, 3H).**

### 1.3 Preparation of compound II-3-a

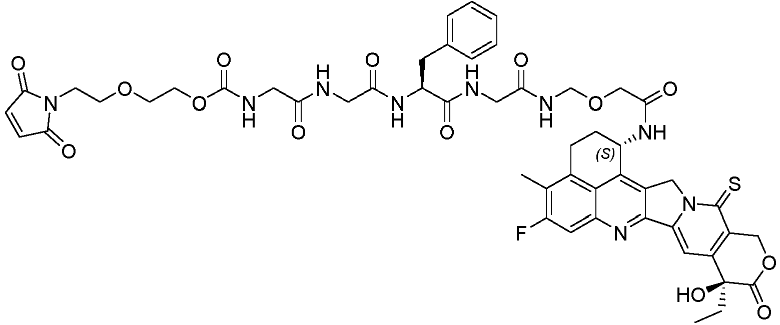

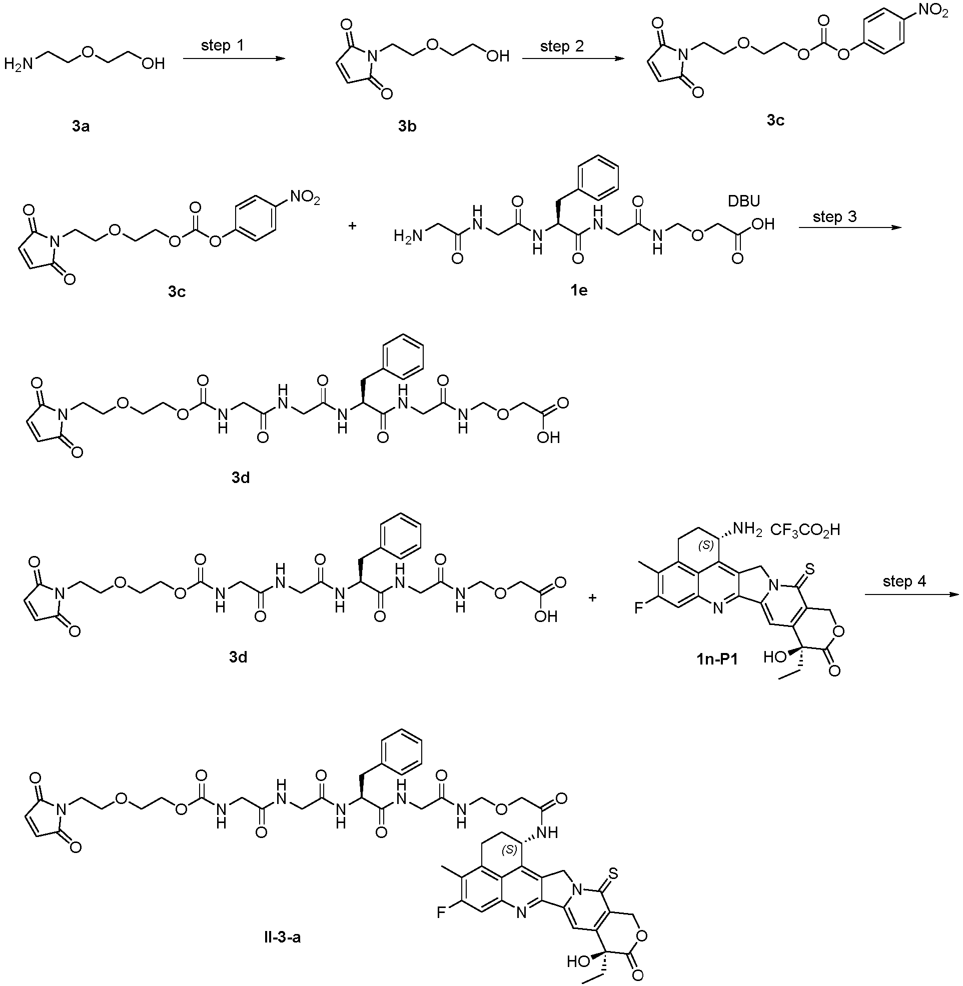

### Step 1

Compound **3a** (10.00 g, 95.11 mmol) was dissolved in *N,N*-dimethylformamide (100 mL), and to the obtained solution maleic anhydride (9.32 g, 95.11 mmol) was added. The reaction mixture was stirred at room temperature for 16 hours under N₂ atmosphere. Water (250 mL) was added to the reaction mixture, and the aqueous phase was extracted with dichloromethane/isopropanol (v:v = 4:1, 200 mL × 3). The combined organic phases were concentrated *in vacuo* to afford crude **3b** (12.00 g).

### Step 2

Crude **3b** (4.00 g, 31.70 mmol) was dissolved in *N,N*-dimethylformamide (60 mL), and to the obtained solution bis(4-nitrophenyl) carbonate (16.40 g, 53.95 mmol) was added. The mixture was heated up to 30°C followed by addition of *N,N-*diisopropylethylamine (4.18 g, 32.40 mmol). The reaction was then stirred for 2 hours. The reaction solution was cooled to room temperature followed by addition of water (600 mL), and the aqueous phase was extracted with dichloromethane (300 mL × 2). The resulting organic phases were combined. The combined organic phases were dried over anhydrous sodium sulfate and then filtrated. The filtrate was concentrated *in vacuo,* and the residue was purified by flash column chromatography on silica gel (ethyl acetate: petroleum ether = 0-100%) to afford compound **3c** (2.40 g) with two-step yield of 13%.

### Step 3

Compound **3c** (400 mg, 1.14 mmol) was dissolved in a mixed solution of acetonitrile (8 mL) and water (16 mL), then compound **1e** (760 mg, 1.13 mmol) was added to the resulting solution, and the resulting reaction mixture was stirred at room temperature for 16 hours under N₂ atmosphere. The reaction mixture was directly purified by prep-HPLC to afford compound **3d** (109 mg) with 15% yield.

### Step 4

To a single-neck flask (10 mL) were added compound **1n-P1** (69 mg, 0.12 mmol), **3d** (110 mg, 0.17 mmol), 1-(3-dimethylaminopropyl)-3-ethylcarbodiimide hydrochloride (44 mg, 0.23 mmol) and *N,N*-dimethylformamide (2.5 mL) successively, then 2,4,6-trimethylpyridine (84 mg, 0.69 mmol) was added dropwise to the obtained mixture. The resulting mixture was stirred at room temperature for 3 hours. The resulting reaction solution was added dropwise to *tert-butyl* methyl ether (75 mL). A solid was precipitated, filtered and collected. The obtained solid was dissolved in a mixed solvent of dichloromethane/methanol (V:V = 6:1, 30 mL). The obtained solution was concentrated *in vacuo,* and the residue was purified by prep-HPLC to afford compound **II-3-a** (45 mg) with 35% yield.

MS-ESI calc. for [M+H]⁺ 1068.4, found: 1068.9.

**¹H NMR (400 MHz, DMSO-*d*₆) δ 8.68 (t, *J =* 6.8 Hz, 1H), 8.62 (d, *J =* 9.2 Hz, 1H), 8.31 (t, *J =* 5.6 Hz, 1H), 8.12 (d, *J =* 8.0 Hz, 1H), 7.99 (t, *J =* 5.6 Hz, 1H), 7.78 (s, 1H), 7.77 (d, *J* = 10.8 Hz, 1H), 7.38 (t, *J* = 6.0 Hz, 1H), 7.27-7.13 (m, 5H), 6.99 (s, 2H), 5.90 (d, *J =* 16.4 Hz, 1H), 5.70-5,61 (m, 1H), 5.51 (s, 1H), 5.46 (d, *J =* 4.4 Hz, 1H), 5.29 (d, *J*** = **19.6 Hz, 1H), 4.67 (d, *J =* 6.4 Hz, 1H), 4.51-4.41 (m, 1H), 4.17-4.04 (m, 2H), 4.02-3.94 (m, 2H), 3.79-3.67 (m, 3H), 3.35-3.20 (m, 1H), 3.20-3.07 (m, 1H), 3.00 (dd, *J =* 14.0, 4.4 Hz, 1H), 2.78-2.65 (m, 1H), 2.37 (s, 3H), 2.25-2.15 (m, 2H), 1.92-1.82 (m, 2H), 0.85 (t, *J* = 7.2 Hz, 3H).**

### 1.4 Preparation of compound II-2-a

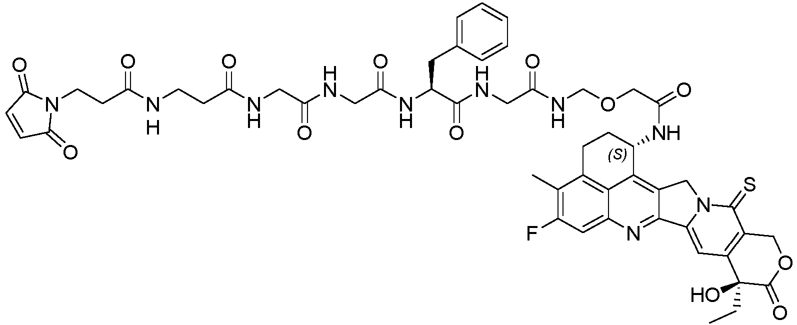

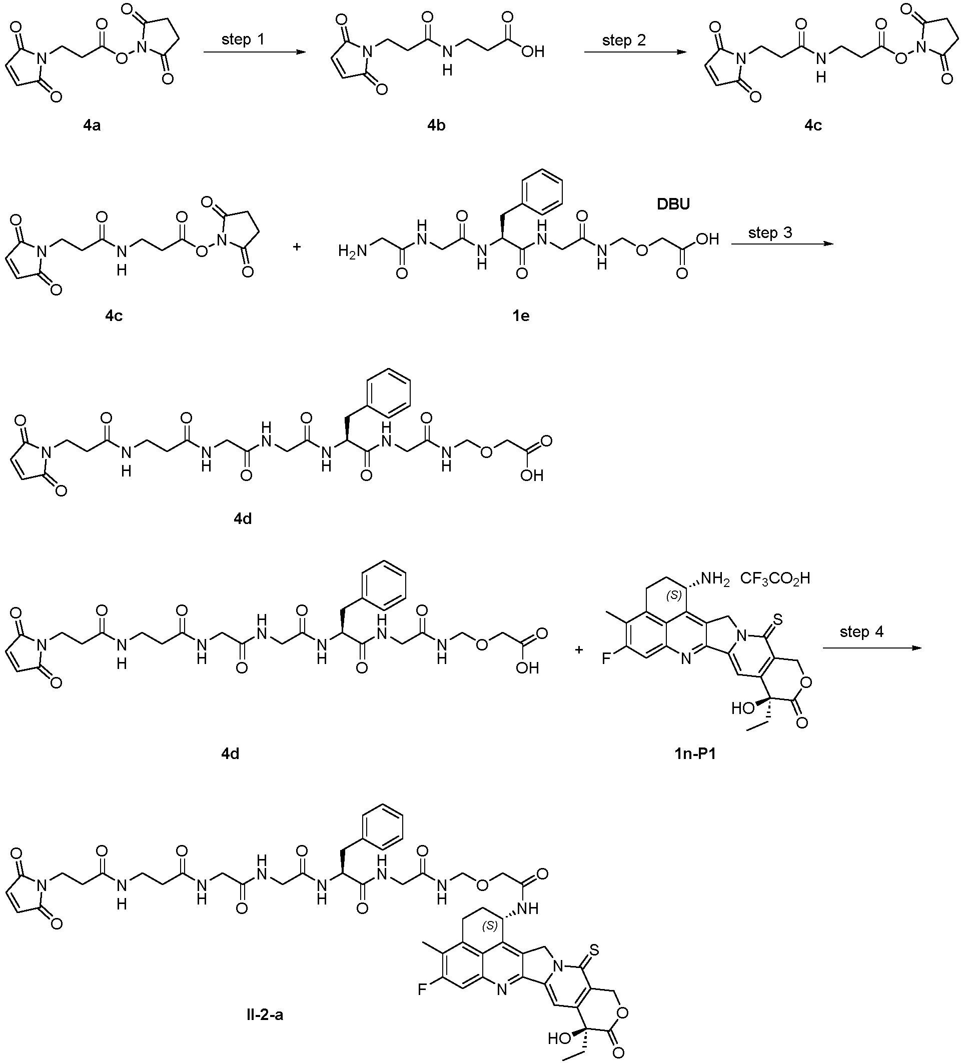

### Step 1

Compound **4a** (2.00 g, 7.52 mmol) and *β*-alanine (0.67 g, 7.52 mmol) were dissolved in acetonitrile (20 mL), then the mixture was cooled to 0°C followed by addition of *N,N-*diisopropylethylamine (1.94 g, 15.03 mmol). After addition was completed, the reaction mixture was recovered to room temperature and stirred for 16 hours. To the reaction mixture water (100 mL) was added, then the reaction mixture was extracted with dichloromethane/isopropanol (v:v = 4:1, 250 mL × 10), and the obtain organic phases were combined. The combined organic phases were concentrated, and the residue was purified by flash column chromatography on silica gel (methanol: dichloromethane = 0-100%) to afford compound **4b** (2.00 g).

### Step 2

Compound **4b** (1.80 g, 7.50 mmol) was dissolved in *N,N-*diisopropylethylamine (8 mL), and to the obtained solution dicyclohexylcarbodiimide (1.85 g, 9.00 mmol) was added. After addition was completed, the reaction mixture was stirred for 30 min followed by addition of *N-*hydroxysuccinimide (0.95 g, 8.25 mmol). The resulting mixture was stirred at room temperature for 16 hours. The reaction mixture was filtered, and the filtrate was purified by prep-HPLC directly to afford compound **4c** (530 mg) with 21% yield.

### Step 3

Compound **4c** (0.50 g, 1.48 mmol) was dissolved in a mixed solvent of acetonitrile (8 mL) and water (16 mL), and to the obtained solution **1e** (0.98 g, 1.50 mmol) was added. The reaction mixture was stirred at room temperature for 16 hours under N₂ atmosphere. The reaction mixture was directly purified by prep-HPLC to afford compound **4d** (42 mg) with 4% yield.

### Step 4

To a single-neck flask (10 mL) were added compound **1n-P1** (26 mg, 0.046 mmol), **4d** (42 mg, 0.065 mmol), 1-(3-dimethylaminopropyl)-3-ethylcarbodiimide hydrochloride (16 mg, 0.083 mmol) and *N,N*-dimethylformamide (1 mL) successively, and then 2,4,6-trimethylpyridine (32 mg,0.26 mmol) was added dropwise to the mixture. The resulting mixture was reacted at room temperature for 3 hours. The reaction solution was added dropwise to *tert-butyl* methyl ether (30 mL). A solid was precipitated, filtered and collected. The obtained solid was dissolved in a mixed solvent of dichloromethane/methanol (V:V = 6:1, 30 mL). The obtained solution was concentrated *in vacuo,* and the residue was purified by prep-HPLC to afford compound **II-2-a** (13 mg) with 26% yield.

MS-ESI calc. for [M+H]⁺1079.4, found: 1079.9.

**¹H NMR (400 MHz, DMSO-*d*₆) δ 8.68 (t, *J =* 6.8 Hz, 1H), 8.63 (d, *J =* 8.8 Hz, 1H), 8.31 (t, *J* = 6.0 Hz, 1H), 8.18 (t, *J =* 7.2 Hz, 1H), 8.10 (d, *J =* 7.2 Hz, 1H), 8.03 (t, *J =* 5.6 Hz, 1H), 7.98 (t, *J =* 5.6 Hz, 1H), 7.82 (d, *J =* 10.8 Hz, 1H), 7.79 (s,1H), 7.28-7.13 (m, 5H), 6.98 (s, 2H), 6.70 (s, 1H), 5.90 (d, *J =* 16.8 Hz, 1H), 5.70-5,60 (m, 1H), 5.56-5.45 (m, 2H), 5.36 (d, *J =* 19.6 Hz, 1H), 4.68 (d, *J =* 6.8 Hz, 1H), 4.53-4.42 (m, 1H), 4.18-4.04 (m, 2H), 3.78-3.64 (m, 5H), 3.62-3.52 (m, 4H), 3.22-3.14 (m, 1H), 3.06-2.97 (m, 1H), 2.80-2.70 (m, 1H), 2.68-2.65 (m, 1H), 2.39 (s, 3H), 2.34-2.16 (m, 6H), 1.92-1.82 (m, 2H), 0.85 (t, *J =* 7.2 Hz, 3H).**

### 1.5 Preparation of compound II-12-b

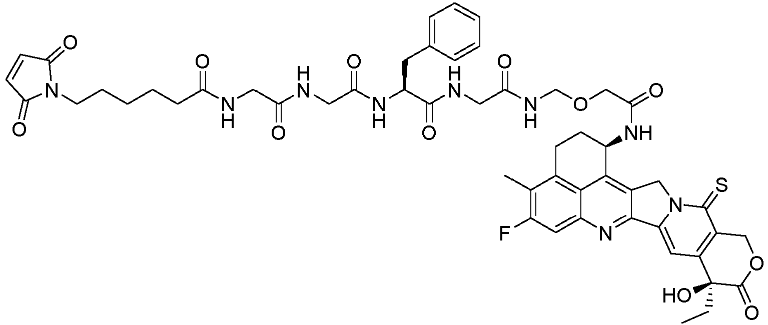

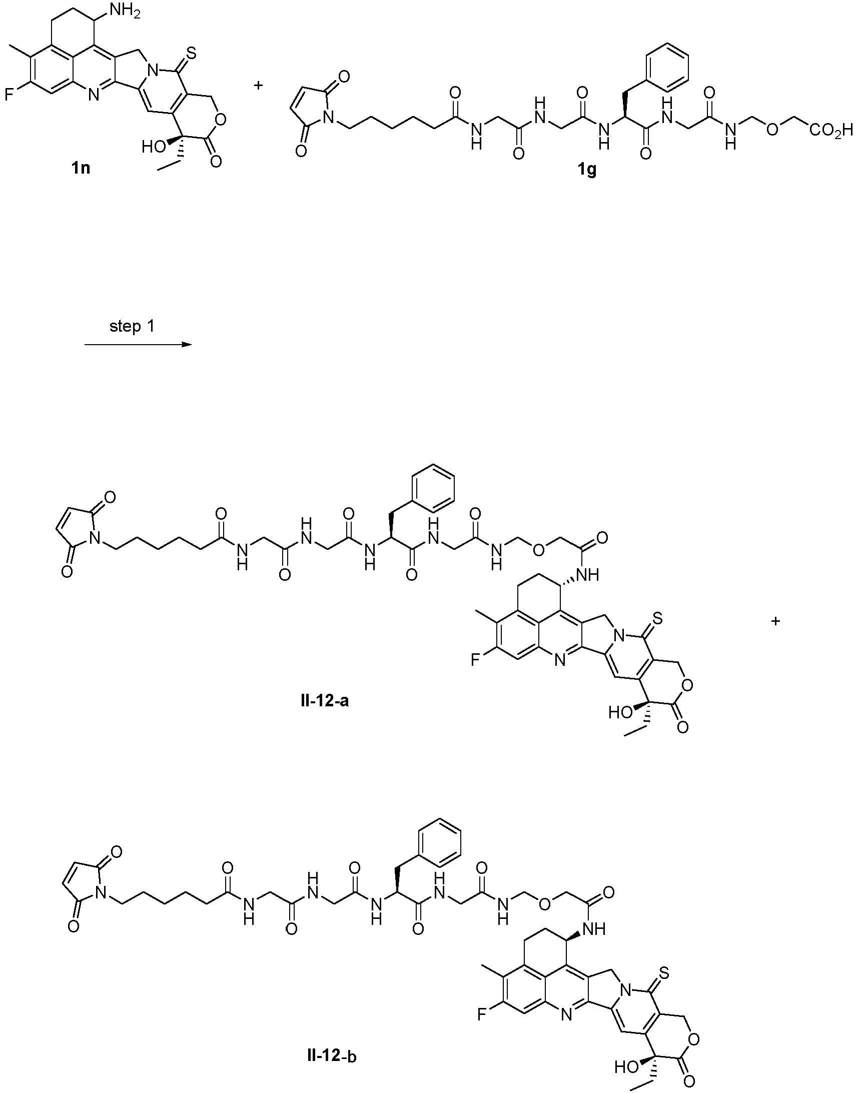

### Step 1

Under N₂ protection and at 0°C, **1n** (24.00 mg, 0.053 mmol) and **1g** (32.78 mg, 0.053 mmol) were dissolved in *N,N*-dimethylformamide (1.5 mL), then a solution of*N,N*-diisopropylethylamine (17.10 mg, 0.0133 mmol) in *N,N*-dimethylformamide (0.4 mL) and a solution of 2-(7-Azabenzotriazol-1-yl)-*N,N,N',N*'-tetramethyluronium hexafluorophosphate (24.20 mg, 0.064 mmol) in *N,N*-dimethylformamide (0.6 mL) were added dropwise successively. After addition was completed, the resulting reaction mixture was maintained at 0°C and reacted for 1 hour. The reaction mixture was purified to afford compound **II-12-a** (16 mg) and **II-12-b** (19 mg) with 64% overall yield.

### II-12-b:

MS-ESI calc. for [M+H]⁺ 1050.4, found: 1050.3.

**¹H NMR (400 MHz, DMSO-*d*6) δ 8.71-8.61 (m, 2H), 8.31 (t, *J =* 4.8 Hz, 1H), 8.12 (d, *J =* 7.2 Hz, 1H), 8.07 (t, *J =* 6.0 Hz, 1H), 8.00 (t, *J =* 5.2 Hz, 1H), 7.82 (dd, *J =* 10.8, 2.0 Hz, 1H), 7.80 (s, 1H), 7.28-7.13 (m, 6H), 6.99 (s, 2H), 6.70 (br s, 1H), 5.92 (d, *J* = 16.8 Hz, 1H), 5.70-5,61 (m, 1H), 5.52 (d, *J* = 17.2 Hz, 2H), 5.37 (d, *J* = 19.6 Hz, 1H), 4.68 (d, *J =* 6.8 Hz, 2H), 4.51-4.41 (m, 1H), 4.18-4.04 (m, 2H), 3.77-3.50 (m, 7H), 3.32-3.20 (m, 1H), 3.20-3.08 (m, 1H), 3.07-2.96 (m, 1H), 2.82-2.71 (m, 1H), 2.39 (s, 3H), 2.26-2.15 (m, 2H), 2.09 (t, *J* = 7.6 Hz, 2H), 1.96-1.82 (m, 2H), 1.52-1.39 (m, 4H), 1.25-1.10 (m, 2H), 0.85 (t, *J =* 7.6 Hz, 3H).**

### 1.6 Preparation of compounds X and Y

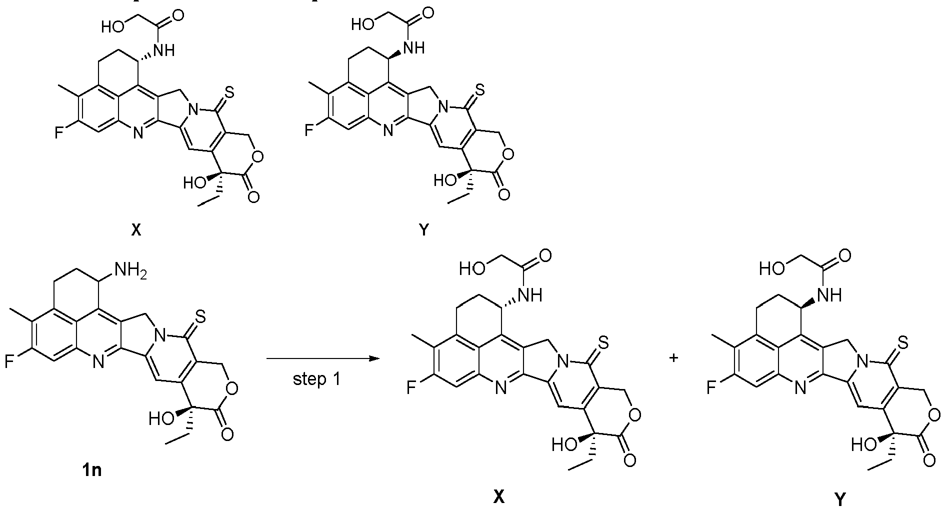

### Step 1

To a single-neck flask (25 mL) were added **1n** (20 mg, 0.04 mmol) and a solution of glycolic acid in *N,N-*dimethylformamide (2.00 mL, 0.032 mmol, 1.2 mg/mL), then the mixture was cooled to 0°C followed by addition of *N,N*-diisopropylethylamine (11 mg, 0.08 mmol) and 2-(7-azabenzotriazol-1-yl)-N,N,N',N'-tetramethyluronium hexafluorophosphate (16 mg, 0.04 mmol) successively. The reaction mixture was reacted for 0.5 hour. Another batch of a solution of glycolic acid in *N,N*-dimethylformamide (1.2 mg/mL, 0.20 mL, 0.003 mmol) and 2-(7-azabenzotriazol-1-yl)-N,N,N',N'-tetramethyluroniumhexafluorophosphate (1 mg) were added, then the reaction mixture was reacted for an additional 1 hour. The reaction mixture was diluted with ethyl acetate (80 mL). The organic phase was washed by 0.5 N HCl_{(aq)} (5 mL × 1), saturated aq. sodium bicarbonate (10 mL × 2) and saturated brine (10 mL × 5) successively. The organic phase was dried over anhydrous sodium sulfate and filtered. The filtrate was concentrated *in vacuo* and the residue was purified by Prep-TLC (methanol: dichloromethane) to afford compounds X (2 mg) and **Y** (5 mg) with 34% overall yield.

### X:

MS-ESI calc. for [M+H]⁺ 510, found: 510.

**¹H NMR (400 MHz, DMSO-*d*₆) δ 8.54 (d, *J =* 8.8 Hz, 1H), 7.82 (d, *J =* 10.8 Hz, 1H), 7.80 (s, 1H), 5.92 (d, *J =* 16.8 Hz, 1H), 5.69 - 5.60 (m, 1H), 5.53 (d, *J =* 20.4 Hz, 1H), 5.52 (d, *J* = 16.4 Hz, 1H), 5.35 (d, *J =* 20.0 Hz, 1H), 4.15 - 3.98 (m, 2H), 3.30 - 3.22 (m, 1H), 3.19 - 3.09 (m, 1H), 2.40 (s, 3H), 2.26 - 2.14 (m, 2H), 1.96 - 1.84 (m, 2H), 0.86 (t, *J =* 7.2 Hz, 3H).**

### Y:

MS-ESI calc. for [M+H]⁺ 510, found: 510.

**¹H NMR (400 MHz, DMSO-*d*₆) δ 8.54 (d, *J =* 9.2 Hz, 1H), 7.81 (d, *J =* 11.2 Hz, 1H), 7.80 (s, 1H), 6.69 (s, 1H), 5.92 (d, *J =* 16.8 Hz, 1H), 5.69 - 5.62 (m, 1H), 5.62 - 5.56 (m, 1H), 5.55 - 5.47 (m, 2H), 5.33 (d, *J =* 20.0 Hz, 1H), 4.16 - 3.98 (m, 2H), 3.30 - 3.20 (m, 1H), 3.16 - 3.07 (m, 1H), 2.39 (s, 3H), 2.26 - 2.15 (m, 2H), 1.95 - 1.85 (m, 2H), 0.86 (t, *J* = 7.2 Hz, 3H).**

### Example 2 Preparation and test of antibody-drug conjugates

### 1. General preparation method

The antibody was dialyzed into 50 mM PB buffer to obtain the antibody intermediate. An appropriate amount of antibody intermediate was taken, and 10 mM tris(2-carboxyethyl)phosphine hydrochloride (TCEP) stock solution, 10 mM diethylenetriaminepentaacetic acid (DTPA) stock solution were added successively, and additional 50 mM PB buffer was added to make the final concentration of antibody in the reaction system to be 5-10 mg/mL, the molar ratio of TCEP to antibody to be (2.2-8.0) : 1.0, and the final concentration of DTPA to be 0.5-1.5 mM. The reaction system was mixed well and then placed in a thermostatic mixer with a reduction reaction temperature of 25±2°C and a rotational speed of 400 rpm to be subjected to a reduction reaction for 1.0-2.5 hours. After completion of reduction reaction, an appropriate amount of 5 mM linker-payload stock solution was sequentially added to each reaction system under ice bath, and the molar ratio of linker-payload to antibody was 4.5-12.0; if the purity of the conjugate product was low, supplemental addition of DMSO and/or arginine hydrochloride maybe considered. The reaction system was mixed well and then placed in a thermostatic mixer with a conjugation reaction temperature of 25 ±2°C and a rotational speed of 400 rpm to be subjected to a conjugation reaction for 0.5-1.5 h. After completion of conjugation reaction, the ADC sample was dialyzed into dialysis solution (10 mM or 20 mM His/His-HCl, pH 6.0±0.2) using ultrafiltration centrifuge tubes to obtain the ADC stock solution, which was aliquoted and stored at -80°C.

### 1.1 Preparation of MH-ADC1

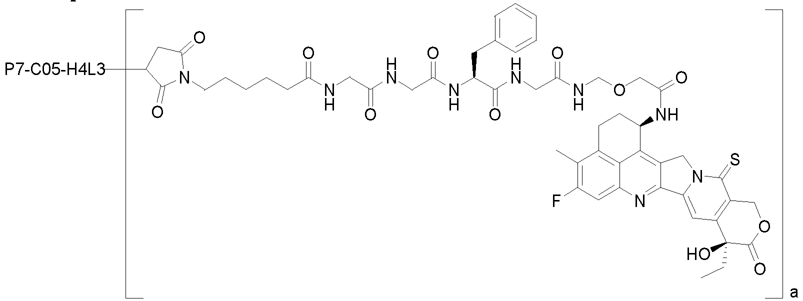

The antibody P7-C05-H4L3 was dialyzed into 50 mM PB (pH 7.4 ± 0.2) buffer to obtain the antibody intermediate. An appropriate amount of antibody intermediate was taken, and an appropriate amount of 10 mM tris(2-carboxyethyl)phosphine hydrochloride (TCEP) stock solution and 10 mM diethylenetriaminepentaacetic acid (DTPA) stock solution were added successively, and additional 50 mM PB (pH 7.4 ± 0.2) buffer was added to make the final concentration of the antibody in the reaction system to be 10 mg/mL, the molar ratio of TCEP to antibody to be 2.4 : 1.0, and the final concentration of DTPA to be 1 mM. The reaction system was mixed well and then placed in a thermostatic mixer with a reduction reaction temperature of 25±2°C and a rotational speed of 400 rpm to be subjected to a reduction reaction for 150 min. The linker-payload **II-12-b** was prepared into a 5 mM stock solution in DMSO. After completion of reduction, an appropriate amount of 5 mM stock solution of linker-payload **II-12-b** was added to the reaction system under ice bath to achieve the molar ratio of linker-payload to antibody was 8.0. The reaction system was mixed well and then placed in a thermostatic mixer with a conjugation reaction temperature of 25 ±2°C and a rotational speed of 400 rpm to be subjected to a conjugation reaction for 60 min. After completion of conjugation reaction, the ADC sample obtained was dialyzed into dialysis solution (20 mM His/His-HCl, pH 6.0±0.2) using an ultrafiltration centrifuge tube to obtain the **MH-ADC1** stock solution, which was aliquoted and stored at -80°C.

### 1.2 Preparation of MH-ADC2

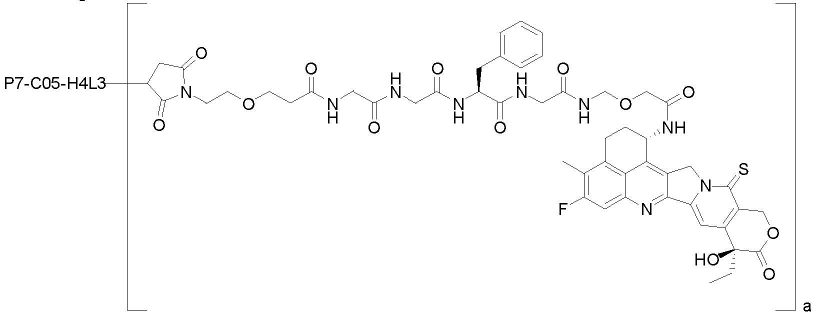

### 1.2.1 Preparation of MH-ADC2-1

The antibody P7-C05-H4L3 was dialyzed into 50 mM PB (pH 7.4±0.2) buffer to obtain the antibody intermediate. An appropriate amount of antibody intermediate was taken, and an appropriate amount of 10 mM tris(2-carboxyethyl)phosphine hydrochloride (TCEP) stock solution and 10 mM diethylenetriaminepentaacetic acid (DTPA) stock solution were added successively, and additional 50 mM PB (pH 7.4 ± 0.2) buffer was added to make the final concentration of the antibody in the reaction system to be 10 mg/mL, the molar ratio of TCEP to antibody to be 2.6 : 1.0, and the final concentration of DTPA to be 1 mM. The reaction system was mixed well and then placed in a thermostatic mixer with a reduction reaction temperature of 25±2°C and a rotational speed of 400 rpm to be subjected to a reduction reaction for 2 h. The linker-payload **II-1-a** was prepared into a 5 mM stock solution in DMSO. After completion of reduction reaction, an appropriate amount of 5 mM stock solution of linker-payload **II-1-a** was added to the reaction system under ice bath to achieve the molar ratio of linker-payload to antibody was 5.5. The reaction system was mixed and then placed in a thermostatic mixer with a conjugation reaction temperature of 25±2°C and a rotational speed of 400 rpm to be subjected to a conjugation reaction for 1 h. After completion of conjugation reaction, the obtained ADC sample was dialyzed into dialysis solution (10 mM His/His-HCl, pH 6.0±0.2) using an ultrafiltration centrifuge tube to obtain the **MH-ADC2-1** stock solution, which was aliquoted and stored at -80°C.

### 1.2.2 Preparation of MH-ADC2-2

The antibody P7-C05-H4L3 was dialyzed into 50 mM PB (pH 7.4 ± 0.2) buffer to obtain the antibody intermediate. An appropriate amount of antibody intermediate was taken, and an appropriate amount of 10 mM tris(2-carboxyethyl)phosphine hydrochloride (TCEP) stock solution and 10 mM diethylenetriaminepentaacetic acid (DTPA) stock solution were added successively, and additional 50 mM PB (pH 7.4 ± 0.2) buffer was added to make the final concentration of the antibody in the reaction system to be 5 mg/mL, the molar ratio of TCEP to antibody to be 2.2 : 1.0, and the final concentration of DTPA to be 1 mM. The reaction system was mixed well and then placed in a thermostatic mixer with a reduction reaction temperature of 25 ±2°C and a rotational speed of 400 rpm to be subjected to a reduction reaction for 1 h. The linker-payload **II-1-a** was prepared into a 5 mM stock solution in DMSO. After completion of reduction reaction, an appropriate amount of 5 mM stock solution of linker-payload **II-1-a** was added to the reaction system under ice bath to achieve the molar ratio of linker-payload to antibody was 5.0. The reaction system was mixed well and then placed in a thermostatic mixer under a conjugation reaction temperature of 25 ±2°C and a rotational speed of 400 rpm to be subjected to a conjugation reaction for 1 h. After completion of conjugation reaction, the obtained ADC sample was dialyzed into dialysis solution (10 mM His/His-HCl, pH 6.0±0.2) using an ultrafiltration centrifuge tube to obtain the **MH-ADC2-2** stock solution, which was divided and stored at -80°C.

### 1.2.3 Preparation of MH-ADC2-3

The antibody P7-C05-H4L3 was dialyzed into 50 mM PB (pH 7.7±0.2) buffer to obtain the antibody intermediate. An appropriate amount of antibody intermediate was taken, and an appropriate amount of 10 mM tris(2-carboxyethyl)phosphine hydrochloride (TCEP) stock solution and 10 mM diethylenetriaminepentaacetic acid (DTPA) stock solution were added successively, and additional 50 mM PB (pH 7.7±0.2) buffer was added to make the final concentration of the antibody in the reaction system to be 10 mg/mL, the molar ratio of TCEP to antibody to be 3.5 : 1.0, and the final concentration of DTPA to be 1 mM. The reaction system was mixed well and then placed in a thermostatic mixer with a reduction reaction temperature of 25±2°C and a rotational speed of 400 rpm to be subjected to a reduction reaction for 2 h. The linker-payload **II-1-a** was prepared into a 5 mM stock solution in DMSO. After completion of reduction reaction, an appropriate amount of 5 mM stock solution of linker-payload **II-1-a** was added to the reaction system under ice bath to achieve the molar ratio of linker-payload to antibody was 7.0. The reaction system was mixed well and then placed in a thermostatic mixer with a conjugation reaction temperature of 25 ±2°C and a rotational speed of 400 rpm to be subjected to a conjugation reaction for 1 h. After completion of conjugation reaction, the obtained ADC sample was dialyzed into dialysis solution (10 mM His/His-HCl, pH 6.0±0.2) using an ultrafiltration centrifuge tube to obtain the **MH-ADC2-3** stock solution, which was aliquoted and stored at -80°C.

### 1.3 Preparation of control ADC1

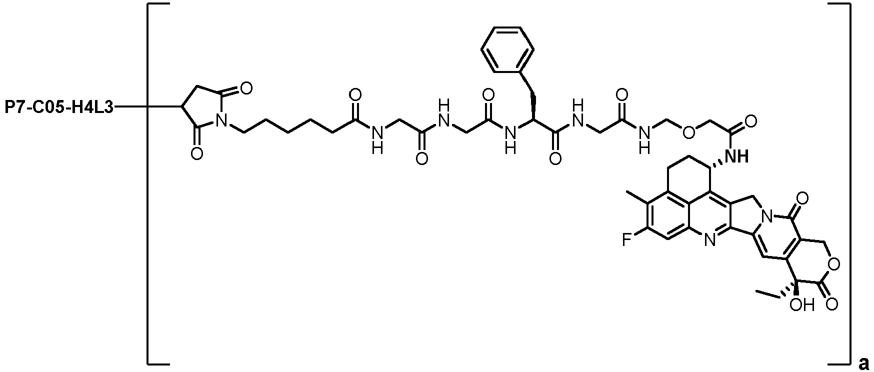

The antibody P7-C05-H4L3 was dialyzed into 50 mM PB (pH 7.4±0.2) buffer to obtain the antibody intermediate. An appropriate amount of antibody intermediate was taken, and an appropriate amount of 10 mM tris(2-carboxyethyl)phosphine hydrochloride (TCEP) stock solution and 10 mM diethylenetriaminepentaacetic acid (DTPA) stock solution were added successively, and additional 50 mM PB (pH 7.4 ± 0.2) buffer was added to make the final concentration of the antibody in the reaction system to be 10 mg/mL, the molar ratio of TCEP to antibody to be 2.6 : 1.0, and the final concentration of DTPA to be 1 mM. The reaction system was mixed and then placed in a thermostatic mixer with a reduction reaction temperature of 25 ±2°C and a rotational speed of 400 rpm to be subjected to a reduction reaction for 150 min. The linker-payload **MC-GGFG-DXd** was prepared into a 5 mM stock solution in DMSO. After completion of reduction reaction, an appropriate amount of 5 mM stock solution of the linker-payload **MC-GGFG-DXd** was added to the reaction system under ice bath to achieve the molar ratio of the linker-payload to the antibody was 8.0. The reaction system was mixed well and then placed in a thermostatic mixer with a conjugation reaction temperature of 25±2°C and a rotational speed of 400 rpm to be subjected to a conjugation reaction for 60 min. After completion of conjugation reaction, the obtained ADC sample was dialyzed into dialysis solution (20 mM His/His-HCl, pH 6.0±0.2) using an ultrafiltration centrifuge tube to obtain the **control ADC1** stock solution, which was aliquoted and stored at -80°C.

### 1.4 Preparation of control ADC2

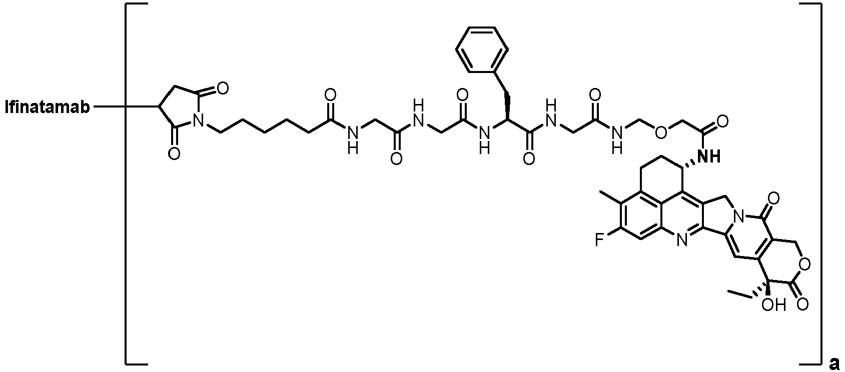

The antibody Ifinatamab was dialyzed into 50 mM PB (pH 7.4±0.2) buffer to obtain the antibody intermediate. An appropriate amount of antibody intermediate was taken, and an appropriate amount of 10 mM tris(2-carboxyethyl)phosphine hydrochloride (TCEP) stock solution and 10 mM diethylenetriaminepentaacetic acid (DTPA) stock solution were added successively, and additional 50 mM PB (pH 7.4 ± 0.2) buffer was added to make the final concentration of antibody in the reaction system to be 5 mg/mL, the molar ratio of TCEP to antibody to be 2.4 : 1.0, and the final concentration of DTPA to be 1 mM. The reaction system was mixed and then placed in a thermostatic mixer with a reduction reaction temperature of 25 ±2°C and a rotational speed of 400 rpm to be subjected to a reduction reaction for 2 h. The linker-payload **MC-GGFG-DXd** was prepared into a 5 mM stock solution in DMSO. After completion of reduction reaction, an appropriate amount of 1 M arginine hydrochloride was added to make the final concentration of arginine hydrochloride in the conjugation reaction system to be 50 mM. An appropriate amount of DMSO and 5 mM stock solution of linker-payload **MC-GGFG-DXd** was added to the reaction system under ice bath to achieve the final concentration of DMSO in reaction system was 15%, and the molar ratio of linker-payload to antibody was 4.5. The reaction system was mixed and then placed in a thermostatic mixer with a conjugation reaction temperature of 25 ±2°C and a rotational speed of 400 rpm to be subjected to a conjugation reaction for 1 h. After completion of conjugation reaction, the obtained ADC sample was dialyzed into dialysis solution (10 mM His/His-HCl, pH 6.0±0.2) using an ultrafiltration centrifuge tube to obtain the **control ADC2** stock solution, which was aliquoted and stored at -80°C.

### 2. Detection of drug-to-antibody ratio of antibody-drug conjugates

The average drug-to-antibody ratio (DAR) of ADC was detected by high performance liquid phase hydrophobic interaction chromatography (HIC-HPLC). After the ADC samples were equilibrated to room temperature and mixed well, the samples were diluted to a target concentration of 5 mg/mL according to the protein concentration using ultra pure water as the diluent and mixed on a vortex mixer for 20s. The diluted sample solution was pipetted into an inserter tube and the sample number was marked on the injection bottle. After equilibration, a high performance liquid phase chromatograph (Agilent, 1260Bio) and a hydrophobic column (TOSOH, TSKgel Butyl-NPR column (2.5) 4.6 mm*35 mm) were used to detected the sample, and the main parameters of the acquisition method were shown in Tables 1 and 2, in which the mobile phase A was formulated as 20 mM sodium phosphate, 1.5 M ammonium sulfate, pH 7.0; mobile phase B was formulated as 20 mM sodium phosphate, 25% isopropanol (v/v), pH 7.0. The HPLC spectrum was integrated, and the percentage of ADCs unconjugated and conjugated with 2, 4, 6, and 8 drugs (DAR0%, DAR2%, DAR4%, DAR6%, and DAR8%, respectively) were obtained based on the integration results, and the average drug-to-antibody ratio was calculated by using the following equation, and the results are shown in Table 3. DAR value = (0×DAR0% + 2×DAR2% + 4×DAR4% + 6×DAR6% + 8×DAR8%) / (DAR0% + DAR2% + DAR4% + DAR6% + DAR8%).

**Table 1. Parameters of the acquisition method of MH-ADC1 and control ADC1**

| | | | |
|---|---|---|---|
| Detection wavelength | | 280 nm | |
| Column temperature | | 25 °C | |
| Flow rate | | 1 mL/min | |
| Injection volume | | 10 µL | |
| Elution gradient | Time (min) | Mobile phase A (%) | Mobile phase B (%) |
| | 0 | 90 | 10 |
| | 15 | 10 | 90 |
| Post-run | | 5 min | |

**Table 2. Parameters of the acquisition method of MH-ADC2-1, MH-ADC2-2, MH-ADC2-3 and control ADC2**

| | | | |
|---|---|---|---|
| Detection wavelength | | 280 nM | |
| Column temperature | | 25 °C | |
| Flow rate | | 1 mL/min | |
| Injection volume | | 10 µL | |
| Elution gradient | Time (min) | Mobile phase A (%) | Mobile phase B (%) |
| | 0 | 90 | 10 |
| | 2 | 90 | 10 |
| | 17 | 10 | 90 |
| Post-run | | 5 min | |

**Table 3. Detection results of drug-to-antibody ratio (DAR)**

| Linker-payload compounds | Antibody | Antibody-drug conjugate | Average drug-to - antibody ratio |
|---|---|---|---|
| II-12-b (1.5 in Example 1) | P7-C05-H4L3 | MH-ADC1 | 3.9 |
| II-1-a (1.2 in Example 1) | P7-C05-H4L3 | MH-ADC2-1 | 4.0 |
| II-1-a (1.2 in Example 1) | P7-C05-H4L3 | MH-ADC2-2 | 3.1 |
| II-1-a (1.2 in Example 1) | P7-C05-H4L3 | MH-ADC2-3 | 5.2 |
| MC-GGFG-DXd | P7-C05-H4L3 | Control ADC 1 | 4.0 |
| MC-GGFG-DXd | Ifinatamab | Control ADC2 | 4.1 |

### Example 3 Inhibition of antibody-drug conjugates against the in vitro proliferation of tumor cells

Human-derived tumor cells in logarithmic growth phase were taken, trypsin-digested and resuspended using fresh complete culture medium, counted and adjusted to an appropriate concentration, and added into 96-well cell culture plate at 100 µL/well. The cell culture plate was placed in a 37°C, 5% CO₂ incubator and incubated overnight. On the next day, different concentrations of ADC samples to be tested (with the highest final concentration of 1 µM, and 4-fold gradient dilution) or buffer control were added to the corresponding wells of the cell culture plate, and continued to be incubated in a CO₂ incubator for 120 h. After equilibrated to room temperature, the test plate was analyzed by using a Cell Titer Glo assay kit (Promega, G7558) and a multifunctional enzyme labeling instrument (Enspire 2300, PerkinElmer) to detect luminescence readings. The cellular inhibition rate was calculated according to the following equation: inhibition rate (%) = (1-(RLU_{ADC} - RLU_{blank})/(RLU_{buffer} - RLU_{blank})) × 100%, wherein, RLU represents Relative Luminescence Units. XLFit was utilized to plot the pharmacodynamic inhibition rate curve and to calculate the IC₅₀ values (see Tables 4 and 5).

The experimental results show that the tested ADCs exhibit excellent inhibitory activities against multiple tumor cell lines with different target expression levels, which are all significantly better than the control ADCs.

**Table 4. Inhibitory activities of ADCs on proliferation of human derived tumor cells in vitro**

| Cell lines | MH-ADC1 IC₅₀ (nM) | Control ADC 1 IC₅₀ (nM) |
|---|---|---|
| Calu-6 | 17.4 | 626.1 |
| NCI-H1975 | 191.3 | >1000 |

**Table 5. Inhibitory activities of ADCs on proliferation of human derived tumor cells in vitro**

| Cell lines | MH-ADC2-1 IC₅₀ (nM) | Control ADC2 IC₅₀ (nM) |
|---|---|---|
| Calu-6 | 0.49 | 125.8 |
| NCI-H1975 | 116.3 | 649.6 |
| NCI-N87 | 74.5 | 287.8 |
| HepG2 | 8.4 | 220.0 |

### Example 4 In vivo pharmacodynamic assay of antibody-drug conjugates in a subcutaneous xenograft tumor model in nude mice with human-derived tumor cells

Human-derived tumor cells in logarithmic growth phase were taken, digested and counted, resuspended in serum-free culture medium, and inoculated subcutaneously in female BALB/c nude mice, with 100 µL of inoculation per animal, to establish a nude mouse xenograft tumor model. After the tumors grew to a measurable range, the long and short diameters of each tumor were measured using vernier calipers, and the tumor volumes were calculated according to the following equation: V=(a×b²)/2, wherein, "a" represents the long diameter of the tumor and "b" represents the short diameter of the tumor.

When the tumor volumes reached an average of about 150 mm³, the mice were randomly grouped according to tumor volume and body weight. Solvent control (saline) or different doses of ADC were injected into the tumor-bearing nude mice via tail vein. The long and short diameters of the tumors were measured twice a week; tumor volumes were counted in each group, and the animal body weights were recorded.

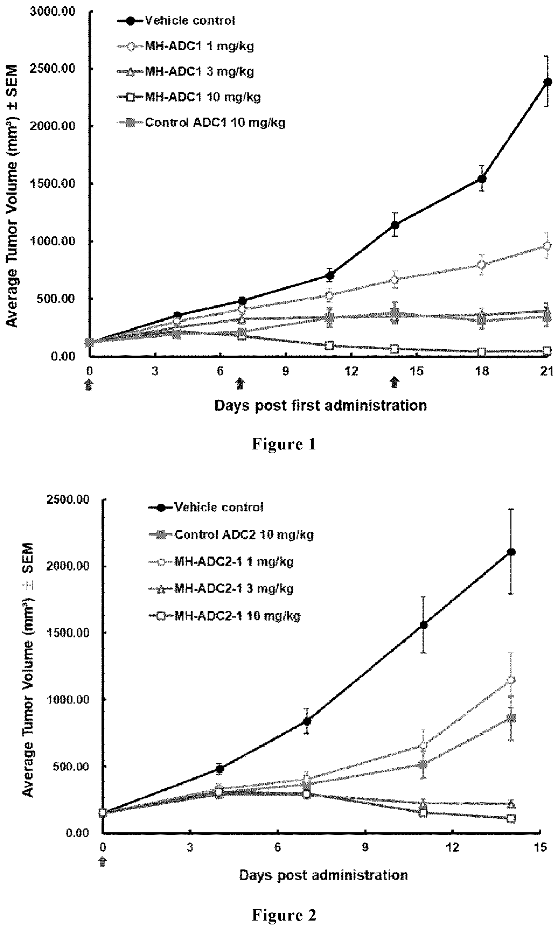

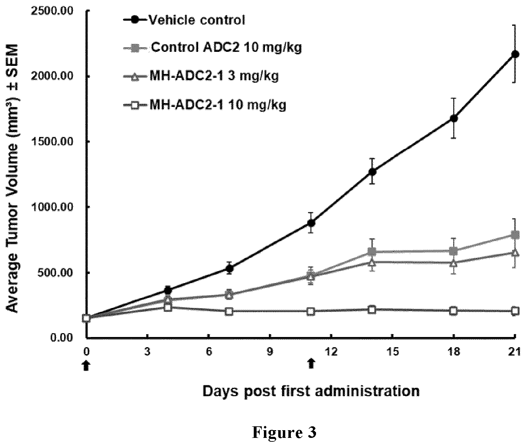

In the efficacy test of MH-ADC1 and control ADC1 in the human lung cancer NCI-H1975 subcutaneous xenograft tumor model, the administration regimen was once a week by tail vein injection, and the results of the test are shown in Figure 1. In the efficacy test of MH-ADC2-1 and control ADC2 in human lung cancer Calu-6 subcutaneous xenograft tumor model, on the day of grouping, the drug was administered via tail vein once, and the results of the test are shown in Figure 2. In the efficacy test of MH-ADC2-1 and control ADC2 in human colorectal cancer HCT116 subcutaneous xenograft tumor model, the drug was administered once via tail vein on the day of grouping and on the 11th day after grouping, respectively, and the results of the test are shown in Figure 3.

The above test results show that the tested ADCs have strong *in vivo* proliferation-inhibition effects in the subcutaneous xenograft tumor model of human derived tumor cells in nude mice and are superior to the control ADCs at the same dose, and in particular, the anti-tumor efficacy of MH-ADC1 and MH-ADC2-1 is significantly better than that of the control ADCs with similar DAR values and the same administration dose.

## Claims

1. An antibody-drug conjugate represented by formula I, a stereoisomer thereof, a prodrug thereof, a pharmaceutically acceptable salt thereof, or a pharmaceutically acceptable solvate thereof,
**Ab-(L-D)ₐ** **I**
wherein,
Ab is an antibody, such as a monoclonal antibody or an antigen-binding fragment thereof;
L is L¹-L²-L³-L⁴;
L¹ is 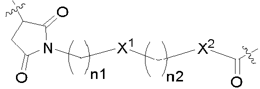
, and the carbonyl carbon end of L¹ is connected to L², and the alkyl carbon end of L¹ is connected to Ab;
n1 is an integer selected from 1 to 5;
preferably, n1 is 1, 2, or 3;
n2 is an integer selected from 1 to 5;
preferably, n2 is 1 or 2;
X¹ is a bond or O;
X² is a bond or O;
L² is a bond or 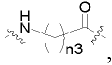 and when L² is 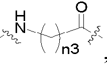 the amino end of L² is connected to L¹, and the carbonyl end of L² is connected to L³;
n3 is an integer selected from 1 to 5;
preferably, n3 is 1, 2, or 3;
more preferably, n3 is 2;
L³ is an amino acid residue or a peptide residue consisting of 2 to 10 amino acid residues, and the amino end of L³ is connected to L² and the carbonyl end of L³ is connected to L⁴;
L⁴ is 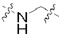 , and the amino end of L⁴ is connected to L³, and the carbon end of L⁴ is connected to D;
D is 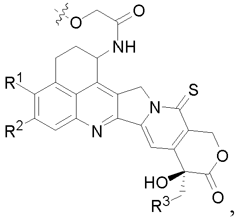 and the O end of D is connected to L⁴, wherein
R¹ is selected from the group consisting of hydrogen, deuterium, halogen, C1-C6 alkyl, deuterated C1-C6 alkyl, and halogenated C1-C6 alkyl;
R² is selected from the group consisting of hydrogen, deuterium, halogen, C1-C6 alkyl, deuterated C1-C6 alkyl, and halogenated C1-C6 alkyl;
R³ is selected from the group consisting of hydrogen, deuterium, halogen, C1-C6 alkyl, deuterated C1-C6 alkyl, and halogenated C1-C6 alkyl;
a is any number between 1 and 10.

2. The antibody-drug conjugate according to claim 1, a stereoisomer thereof, a prodrug thereof, a pharmaceutically acceptable salt thereof, or a pharmaceutically acceptable solvate thereof, wherein L¹ is selected from the group consisting of 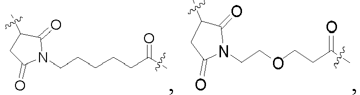 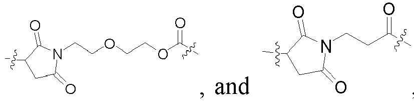 , and the carbonyl carbon end of L¹ is connected to L², and the alkyl carbon end of L¹ is connected to Ab;
preferably, L¹ is selected from the group consisting of 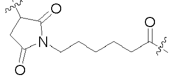 and 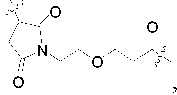 and the carbonyl carbon end of L¹ is connected to L², and the alkyl carbon end of L¹ is connected to Ab.

3. The antibody-drug conjugate according to any one of claims 1-2, a stereoisomer thereof, a prodrug thereof, a pharmaceutically acceptable salt thereof, or a pharmaceutically acceptable solvate thereof, wherein L³ is a peptide residue consisting of 2-4 (preferably 4) amino acid residues, and the amino end connected to L² and the carbonyl end connected to L⁴, preferably, the amino acid is selected from the group consisting of glycine, phenylalanine, valine, alanine, lysine, citrulline, serine, glutamic acid, and aspartic acid, and more preferably, the amino acid is selected from the group consisting of glycine and phenylalanine;
preferably, L³ is glycine-glycine-phenylalanine-glycine (Gly-Gly-Phe-Gly), and the amino end is connected to L², and the carbonyl end is connected to L⁴;
more preferably, L³ is 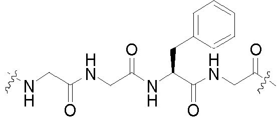 , and the amino end is connected to L² and the carbonyl end is connected to L⁴.

4. The antibody-drug conjugate according to any one of claims 1-3, a stereoisomer thereof, a prodrug thereof, a pharmaceutically acceptable salt thereof, or a pharmaceutically acceptable solvate thereof, wherein R¹ is selected from the group consisting of hydrogen, halogen and C1-C6 alkyl;
preferably, R¹ is C1-C6 alkyl;
more preferably, R¹ is methyl;
or, R² is selected from the group consisting of hydrogen, halogen, and C1-C6 alkyl;
preferably, R² is halogen;
more preferably, R² is fluorine;
or, R³ is selected from the group consisting of hydrogen, halogen, and C1-C6 alkyl;
preferably, R³ is C1-C6 alkyl;
more preferably, R³ is methyl.

5. The antibody-drug conjugate according to any one of claims 1-4, a stereoisomer thereof, a prodrug thereof, a pharmaceutically acceptable salt thereof, or a pharmaceutically acceptable solvate thereof, wherein L is selected from the group consisting of: 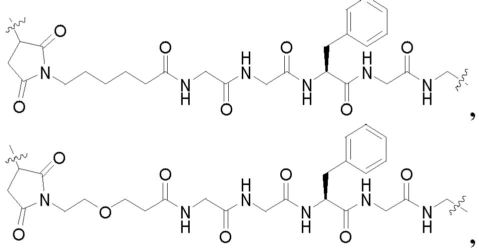 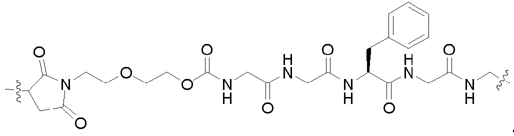 , and 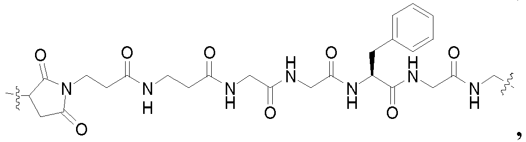
wherein the left carbon end of L is connected to Ab, and the right carbon end of L is connected to D;
or, D is 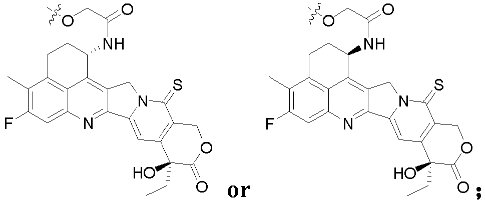
or, L-D as a whole is selected from the group consisting of: 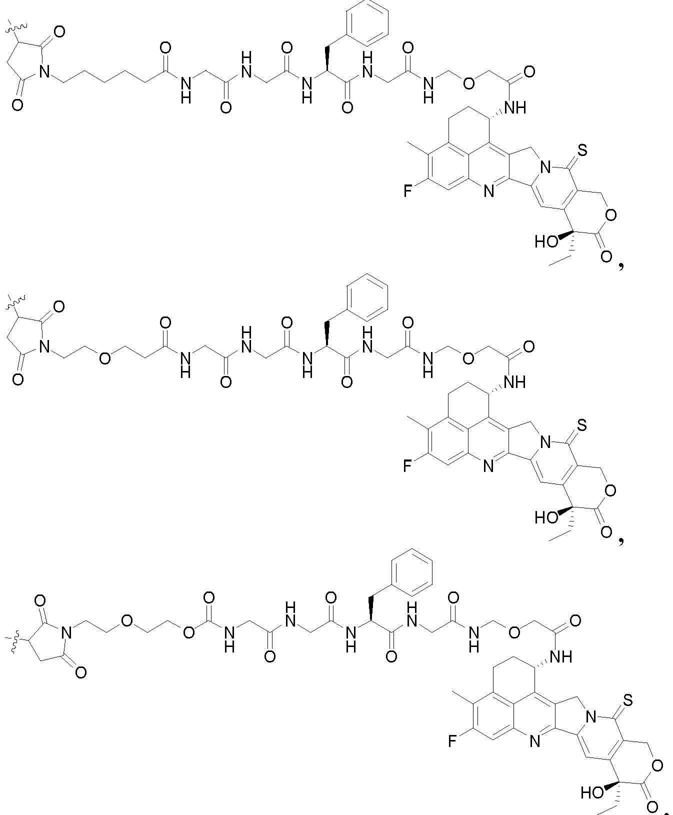 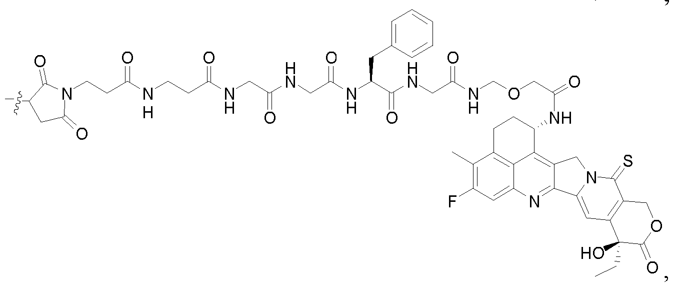 and

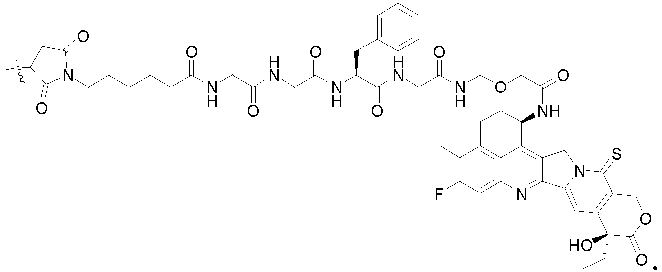

6. The antibody-drug conjugate according to any one of claims 1-5, a stereoisomer thereof, a prodrug thereof, a pharmaceutically acceptable salt thereof, or a pharmaceutically acceptable solvate thereof, wherein the antibody-drug conjugate represented by formula I is selected from the group consisting of:
| **No.** | **Structure** |
|---|---|
| **MH-ADC1** | 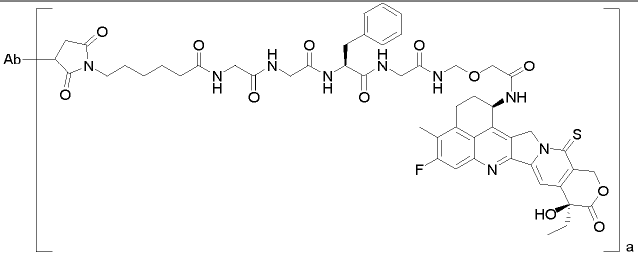 |
| **MH-ADC2** | 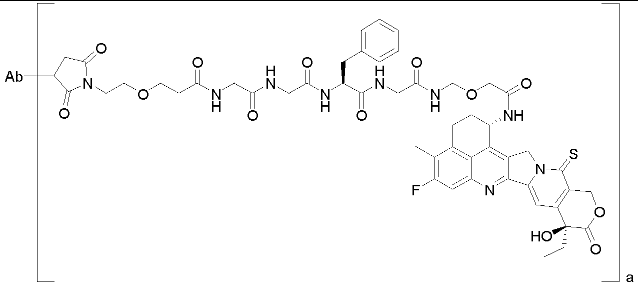 |
| **MH-ADC3** | 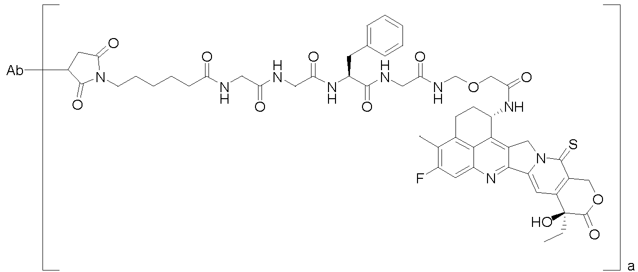 |
| **MH-ADC4** | 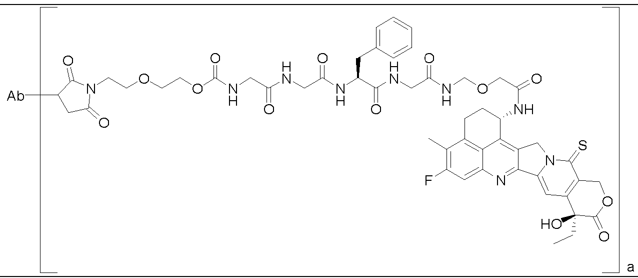 |
| **MH-ADC5** | 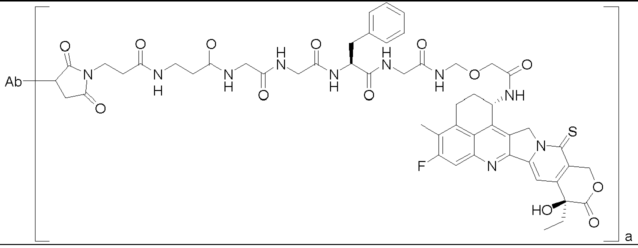 |
wherein Ab and a are defined as described in claim 1.

7. The antibody-drug conjugate according to any one of claims 1-6, a stereoisomer thereof, a prodrug thereof, a pharmaceutically acceptable salt thereof, or a pharmaceutically acceptable solvate thereof, wherein each a is independently any number between 1 and 8;
preferably, each a is independently any number between 3 and 8;
more preferably, each a is independently any number between 3 and 6.

8. The antibody-drug conjugate according to any one of claims 1-7, a stereoisomer thereof, a prodrug thereof, a pharmaceutically acceptable salt thereof, or a pharmaceutically acceptable solvate thereof, wherein Ab is an anti-B7-H3 antibody or an antigen-binding fragment thereof;
preferably, the anti-B7-H3 antibody or the antigen-binding fragment thereof comprises:
(a) three heavy chain variable region (VH) complementarity determining regions (CDRs) as described below:
**(i)** a VH CDR1, having a CDR1 sequence contained in a VH as shown in SEQ ID NO: 8, or a sequence having one or several amino acids substituted, deleted, or added (e.g., substitution, deletion, or addition of one or two amino acids) as compared to the CDR1 sequence contained in the VH;
**(ii)** a VH CDR2, having a CDR2 sequence contained in a VH as shown in SEQ ID NO: 8, or a sequence having one or several amino acids substituted, deleted, or added (e.g., substitution, deletion, or addition of one or two amino acids) as compared to the CDR2 sequence contained in the VH; and
(iii) a VH CDR3, having a CDR3 sequence contained in a VH as shown in SEQ ID NO: 8, or a sequence having one or several amino acids substituted, deleted, or added (e.g., substitution, deletion, or addition of one or two amino acids) as compared to the CDR3 sequence contained in the VH;
and/or
**(b)** three light chain variable region (VL) CDRs as described below:
**(iv)** a VL CDR1, having a CDR1 sequence contained in a VL as shown in SEQ ID NO: 12, or a sequence having one or several amino acids substituted, deleted, or added (e.g., substitution, deletion, or addition of one or two amino acids) as compared to the CDR1 sequence contained in the VL;
**(v)** a VL CDR2, having a CDR2 sequence contained in a VL as shown in SEQ ID NO: 12, or a sequence having one or several amino acids substituted, deleted, or added (e.g., substitution, deletion, or addition of one or two amino acids) as compared to the CDR2 sequence contained in the VL; and
**(vi)** a VL CDR3, having a CDR3 sequence contained in a VL as shown in SEQ ID NO: 12, or a sequence having one or several amino acids substituted, deleted, or added (e.g., substitution, deletion, or addition of one or two amino acids) as compared to the CDR3 sequence contained in the VL;
preferably, the substitution (or substituted) described in any one of **(i)** - **(vi)** is a conservative substitution;
preferably, the CDR1, CDR2, and CDR3 contained in the heavy chain variable region (VH), and/or the CDR1, CDR2, and CDR3 contained in the light chain variable region (VL) are defined by Kabat, Chothia, or IMGT numbering systems;
preferably, the amino acid sequences of heavy chain variable regions CDR1, CDR2, and CDR3 of the anti-B7-H3 antibody or the antigen-binding fragment thereof are shown in SEQ ID NO: 5, SEQ ID NO: 6, and SEQ ID NO: 7, respectively; the amino acid sequences of the light chain variable regions CDR1, CDR2, and CDR3 of the anti-B7-H3 antibody or the antigen-binding fragment thereof are shown in SEQ ID NO: 9, SEQ ID NO: 10, and SEQ ID NO: 11, respectively;
more preferably, the amino acid sequence of heavy chain variable region of the anti-B7-H3 antibody or the antigen-binding fragment thereof is shown in SEQ ID NO: 8, and the amino acid sequence of light chain variable region of the anti-B7-H3 antibody or the antigen-binding fragment thereof is shown in SEQ ID NO: 12;
most preferably, the amino acid sequence of heavy chain of the anti-B7-H3 antibody is shown in SEQ ID NO:1, and the amino acid sequence of light chain of the anti-B7-H3 antibody is shown in SEQ ID NO:2.

9. A pharmaceutical composition, comprising the antibody-drug conjugate according to any one of claims 1-8, a stereoisomer thereof, a prodrug thereof, a pharmaceutically acceptable salt thereof, or a pharmaceutically acceptable solvate thereof, and optionally one or more pharmaceutical excipients.

10. A use of the antibody-drug conjugate according to any one of claims 1-8, a stereoisomer thereof, a prodrug thereof, a pharmaceutically acceptable salt thereof, or a pharmaceutically acceptable solvate thereof, or the pharmaceutical composition according to claim 9 in the manufacture of a medicament for the treatment and/or prevention of a disease,
preferably, the disease is a disease associated with abnormal B7-H3 expression or function;
preferably, the disease is a cancer or an autoimmune disease;
more preferably, the disease is selected from the group consisting of lung cancer (e.g., undifferentiated carcinoma of lung, small cell lung cancer, non-small cell lung cancer), esophageal cancer, gastric cancer, liver cancer, melanoma, prostate cancer, ovarian cancer, endometrial cancer, cervical cancer, breast cancer, head and neck cancer, colorectal cancer, pancreatic cancer, thyroid cancer, sarcoma, cholangiocarcinoma, glioblastoma, and neuroblastoma.
